(19)

Europäisches
Patentamt
European
Patent Office
Office européen
des brevets

(11) **EP 2 832 289 A1**

(12) **EUROPEAN PATENT APPLICATION**
published in accordance with Art. 153(4) EPC

(43) Date of publication:
**04.02.2015 Bulletin 2015/06**

(21) Application number: **13769478.2**

(22) Date of filing: **26.03.2013**

(51) Int Cl.:
***A61B 5/0245*** *(2006.01)*     ***A61B 5/0205*** *(2006.01)*

(86) International application number:
**PCT/JP2013/002063**

(87) International publication number:
**WO 2013/145728 (03.10.2013 Gazette 2013/40)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**

(30) Priority: **29.03.2012 JP 2012076476**

(71) Applicant: **Seiko Epson Corporation
Shinjuku-ku,
Tokyo 163-0811 (JP)**

(72) Inventor: **NAKAMURA, Hidenori
Suwa-shi
Nagano 392-8502 (JP)**

(74) Representative: **Hoffmann Eitle
Patent- und Rechtsanwälte PartmbB
Arabellastraße 30
81925 München (DE)**

(54) **PULSE DETECTOR, ELECTRONIC DEVICE, AND PROGRAM**

(57)     A pulse detector, an electronic apparatus, a program, and the like capable of reducing power consumption according to the determination result of an exercise state, or the like are to be provided.

A pulse detector 100 includes a pulse information calculation unit 120 which calculates pulse information on the basis of a pulse wave detection signal from a pulse wave detection unit 10 having a pulse wave sensor 11 and a body motion detection signal from a body motion detection unit 20 having a body motion sensor, a determination unit 112 which determines the exercise state of a subject, and a control unit 150 which controls at least one of a pulse wave sensing operation in the pulse wave detection unit 10, a body motion sensing operation in the body motion detection unit 20, and a calculation processing rat in the pulse information calculation unit 120 on the basis of the determination result of the exercise state in the determination unit 112.

FIG. 5

EP 2 832 289 A1

**Description**

Technical Field

The present invention relates to a pulse detector, an electronic apparatus, a program, and the like.

Background Art

In the related art, an electronic apparatus including a pulse detector, such as a pulsimeter, has come into wide use. The pulse detector is a device which detects a pulse resulting from heartbeat of a human body, and is a device which detects a signal resulting from heartbeat on the basis of a signal from a pulse wave sensor mounted on an arm, a palm, or a finger, or the like.

As the pulse wave sensor, for example, a photoelectric sensor is used. In this case, a method which detects reflected light or transmitted light of light irradiated onto a living body by the photoelectric sensor, or the like is considered. Since the amount of absorption and the amount of reflection of irradiated light in the living body differ depending on the blood flow rate in a blood vessel, sensor information (pulse wave sensor signal) detected by the photoelectric sensor becomes a signal corresponding to the blood flow rate or the like, and the signal is analyzed, thereby acquiring information relating to a pulse.

However, noise (body motion noise) which is generated by the effect of body motion of a human body is mixed in the pulse wave sensor signal. Accordingly, PTL 1 discloses a method which performs noise reduction processing for reducing body motion noise or the like without using the pulse wave sensor signal directly to detect a pulse signal.

Citation List

Patent Literature

PTL 1: JP-A-60-259239

Summary of Invention

Technical Problem

In recent years, there has been a progress in reduction in size and weight of a pulse detector, such as a wristwatch-type pulsimeter, and an electronic apparatus including the pulse detector. Accordingly, the use which assumes continuous operation for about half a day to several days is considered. When long-term continuous operation is taken into consideration, in the above-described wristwatch-type pulsimeter or the like, there is a major problem in that battery capacity is limited due to limited size or weight. However, the method of PTL 1 does not take into consideration reduction in power consumption or the like, and it is not appropriate for the use of the pulsimeter or the like.

According to several aspects of the invention, it is possible to provide a pulse detector, an electronic apparatus, a program, and the like capable of reducing power consumption according to the determination result of an exercise state, or the like.

Solution to Problem

A pulse detector according to an aspect of the invention includes a pulse information calculation unit which calculates pulse information on the basis of a pulse wave detection signal from a pulse wave detection unit having a pulse wave sensor and a body motion detection signal from a body motion detection unit having a body motion sensor, a determination unit which determines the exercise state of a subject, and a control unit which controls at least one of a pulse wave sensing operation in the pulse wave detection unit, a body motion sensing operation in the body motion detection unit, and a calculation processing rate in the pulse information calculation unit on the basis of the determination result of the exercise state in the determination unit.

In the aspect of the invention, at least one of the pulse wave sensing operation, the body motion sensing operation, and the calculation processing rate of the pulse information is controlled on the basis of the determination result of the exercise state. Accordingly, it is possible to achieve reduction in operation load or the like according to the exercise state, and to achieve reduction in power consumption or the like.

In the aspect of the invention, the determination unit may determine whether the subject is exercising or in living activity, and the control unit may perform at least one of control for lowering the operation rate of the pulse wave sensing operation, control for lowering the operation rate of the body motion sensing operation, and control for lowering the calculation processing rate when the determination unit determines that the subject is in living activity compared to when it is determined that the subject is exercising.

With this, it becomes possible to reduce power consumption or the like in living activity compared to during exercising.

In the aspect of the invention, the determination unit may determine whether the subject is in living activity or sleeping, and the control unit may perform at least one of control for lowering the operation rate of the pulse wave sensing operation, control for lowering the operation rate of the body motion sensing operation, and control for lowering the calculation processing rate when the determination unit determines that the subject is sleeping compared to when it is determined that the subject is in living activity.

With this, it becomes possible to reduce power consumption or the like during sleeping compared to in living activity.

In the aspect of the invention, the determination unit may determine whether the subject is exercising or sleeping, and the control unit may perform at least one of control for lowering the operation rate of the pulse wave sensing operation, control for lowering the operation rate of the body motion sensing operation, and control for lowering the calculation processing rate when the determination unit determines that the subject is sleeping compared to when it is determined that the subject is exercising.

With this, it becomes possible to reduce power consumption or the like during sleeping compared to during exercising.

In the aspect of the invention, the control unit may control calculation processing contents in the pulse information calculation unit on the basis of the determination result of the exercise state in the determination unit.

With this, it becomes possible to control the calculation processing contents of the pulse information on the basis of the determination result of the exercise state.

In the aspect of the invention, the pulse information calculation unit may include a noise reduction unit which performs noise reduction processing for the pulse wave detection signal, and the control unit may control the contents of the noise reduction processing in the noise reduction unit on the basis of the determination result of the exercise state.

With this, it becomes possible to control the noise reduction processing contents on the basis of the determination result of the exercise state.

In the aspect of the invention, the determination unit may determine whether the subject is exercising or in living activity, and the noise reduction unit may have a first filter which performs enhancement filter processing, and a second filter which performs the noise reduction processing on the basis of the body motion detection signal, may perform the noise reduction processing by the first filter and the second filter when it is determined that the subject is exercising, and may perform the noise reduction processing by the first filter when it is determined that the subject is in living activity.

With this, it becomes possible to control the noise reduction filter operation, thereby controlling the noise reduction processing contents.

In the aspect of the invention, the determination unit may perform the determination of the exercise state on the basis of the body motion detection signal in a required exercise state determination period.

With this, it becomes possible to perform exercise state determination or the like based on the body motion detection signal.

In the aspect of the invention, the determination unit may acquire an intermediate result corresponding to the exercise state of one of exercising, in living activity, and sleeping on the basis of the body motion detection signal in the exercise state determination period and may determine the exercise state on the basis of one or a plurality of intermediate results.

With this, it becomes possible to perform exercise state determination taking a plurality of determination results or the like into consideration.

In the aspect of the invention, when it is determined that the subject is exercising, the determination unit may determine that the subject is in living activity when the intermediate result corresponding to the exercise state other than exercising is acquired N (where N is an integer equal to or greater than 2) times, and when it is determined that the subject is in living activity, the determination unit may determine that the subject is exercising when the intermediate result corresponding to exercising is acquired once or M (where M is an integer which satisfies M < N and is equal to or greater than 2) times.

With this, in regard to transition from exercising to in living activity, it becomes possible to provide a smooth transition in a transition direction, or the like.

In the aspect of the invention, when it is determined that the subject is in living activity, the determination unit may determine that the subject is sleeping when the intermediate result corresponding to sleeping is acquired P (where P is an integer equal to or greater than 2) times, and when it is determined that the subject is sleeping, the determination unit may determine that the subject is in living activity when the intermediate result corresponding to in living activity is acquired once or Q (where Q is an integer which satisfies Q < P and is equal to or greater than 2) times.

With this, in regard to transition between in living activity and sleeping, it becomes possible to provide a difference in easiness of the transition in a transition direction, or the like.

In the aspect of the invention, the determination unit may count a frequency in which the signal value of the body motion detection signal exceeds a required signal threshold value in the exercise state determination period and may determine that the subject is sleeping when the frequency is smaller than a required frequency threshold value.

With this, it becomes possible to distinguish between sleeping and other exercise states on the basis of comparison processing of the signal value of the body motion detection signal and the signal threshold value and comparison processing of the frequency in which the signal value exceeds the signal threshold value and the frequency threshold value.

In the aspect of the invention, when it is determined that the subject is not sleeping, the determination unit may perform determination on the presence/absence of periodicity of the body motion detection signal, may determine that the subject is exercising when there is periodicity, and may determine that the subject is in living activity when there is no periodicity.

With this, it becomes possible to distinguish between exercising and in living activity on the basis of the presence/absence of periodicity of the body motion detection signal.

In the aspect of the invention, the determination unit may determine whether the subject is exercising or in living activity, when the determination unit determines that the subject is in living activity, the control unit may perform control for setting the operation rate of the pulse wave sensing operation to the operation rate whose ratio to the operation rate of the pulse wave sensing operation when it is determined that the subject is exercising is R1 (where R1 is a real number which satisfies 0 < R1 < 1), and the control unit may perform control for setting the operation rate of the body motion sensing operation to the operation rate whose ratio to the operation rate of the body motion sensing operation when it is determined that the subject is exercising is R2 (where R2 is a real number which satisfies 0 < R1 < R2 ≤1).

With this, it becomes possible to provide the difference in the degree of lowering of the operation rate based on exercising when it is determined to be in living activity between the pulse wave sensing operation and the body motion sensing operation, or the like.

In the aspect of the invention, the body motion detection unit may have an exercise state determination sensor and a body motion noise reduction sensor as the body motion sensor, and the control unit may control the sensing operation of the body motion noise reduction sensor on the basis of the determination result of the exercise state in the determination unit.

With this, it becomes possible to control the sensing operation of the body motion noise reduction sensor on the basis of the determination result of the exercise state.

In the aspect of the invention, the control unit may perform control for excluding the sensing operation of the exercise state determination sensor from a control target based on the determination result of the exercise state in the determination unit.

With this, it becomes possible to exclude the exercise state determination sensor from the control target based on the determination result of the exercise state.

A pulse detector according to another aspect of the invention includes a pulse information calculation unit which calculates pulse information on the basis of a pulse wave detection signal from a pulse wave detection unit having a pulse wave sensor and a body motion detection signal from a body motion detection unit having a body motion sensor, a determination unit which determines the exercise state of a subject, and a control unit which performs control on the basis of the determination result of the exercise state in the determination unit, in which the pulse information calculation unit includes a noise reduction unit which performs noise reduction processing for the pulse wave detection signal, and the control unit controls the contents of the noise reduction processing in the noise reduction unit on the basis of the determination result of the exercise state.

An electronic apparatus according to still another aspect of the invention includes the above-described pulse detector, the above-described pulse wave detection unit, and the above-described body motion detection unit.

According to still another aspect of the invention, there is provided a program which causes a computer to function as the above-described respective units.

Brief Description of Drawings

[Fig. 1] Fig. 1 shows a basic configuration example of an electronic apparatus including a pulse detector of this embodiment.

[Fig. 2] Fig. 2 shows a configuration example of a body motion noise reduction unit using an adaptive filter.

[Fig. 3] Figs. 3(A) to Fig. 3(C) show examples of the waveforms and frequency spectrums of a pulse wave detection signal, a body motion detection signal, and a signal after body motion noise reduction processing based on these signals.

[Fig. 4] Figs. 4(A) and 4(B) show an example of an electronic apparatus including a pulse detector.

[Fig. 5] Fig. 5 shows a detailed configuration example of the electronic apparatus including the pulse detector of this embodiment.

[Fig. 6] Fig. 6 shows a configuration example of a noise reduction unit.

[Fig. 7] Figs. 7(A) and 7(B) are diagrams illustrating periodicity of an acceleration detection value.

[Fig. 8] Fig. 8 shows an example of a control method based on the determination result of an exercise state.

[Fig. 9] Fig. 9 shows a specific example of the effect of reducing power consumption through control based on the determination result of an exercise state.

[Fig. 10] Fig. 10 is a timing chart showing the operation of an acceleration sensor and a determination unit.

[Fig. 11] Fig. 11 is a timing chart showing the operation of a pulse wave sensor, a pulse information calculation unit, and a display unit during exercising.

[Fig. 12] Fig. 12 is a timing chart showing the operation of a pulse wave sensor, a pulse information calculation unit, and a display unit in living activity.

[Fig. 13] Fig. 13 is a timing chart showing the operation of a pulse wave sensor, a pulse information calculation unit, and a display unit during sleeping.

[Fig. 14] Figs. 14 (A) and 14 (B) are diagrams illustrating transition between exercise states.

[Fig. 15] Fig. 15 is a flowchart illustrating processing of this embodiment.

[Fig. 16] Fig. 16 is a flowchart illustrating 1 Hz interrupt processing.

[Fig. 17] Figs. 17(A) and 17(B) are flowcharts illustrating timer processing.

[Fig. 18] Figs. 18(A) and 18(B) are flowcharts illustrating timer processing.

[Fig. 19] Fig. 19 is a diagram illustrating the relationship between an exercise state and timer processing.

Description of Embodiments

Hereinafter, an embodiment will be described. The embodiment described below is not intended to unduly limit the content of the invention described in the appended claims. It is not always the case that all configurations described in the embodiment are the essential constituent elements of the invention.

1. Configuration example of electronic apparatus including pulse detector

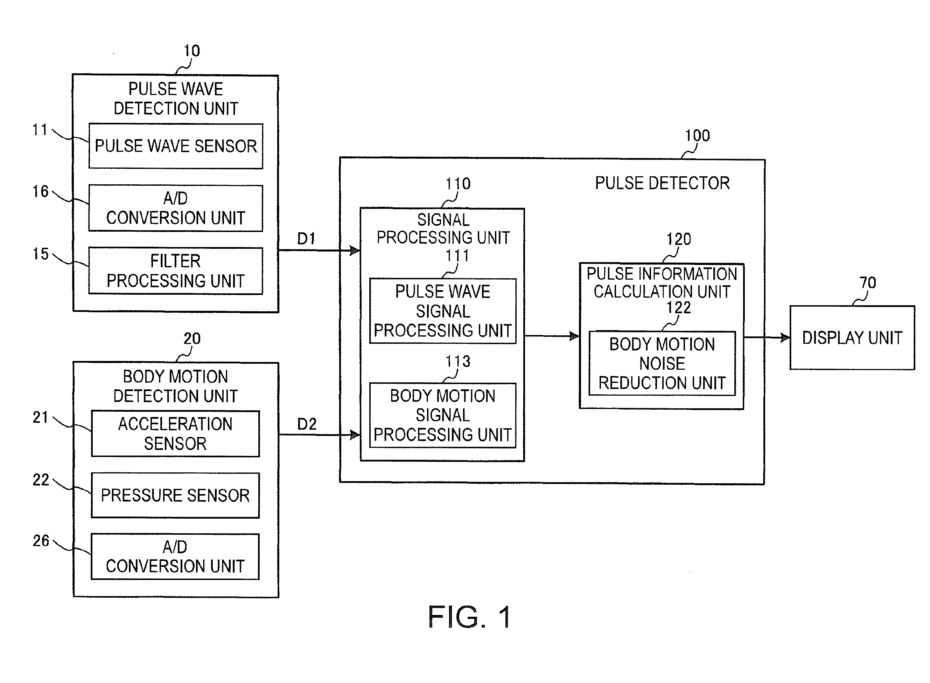

First, a basic configuration example of a pulse detector of the embodiment and an electronic apparatus (in a narrow sense, a pulsimeter) including the pulse detector will be described referring to Fig. 1. Fig. 1 shows an example of a pulse detector and an electronic apparatus, and the configuration included in the pulse detector of the embodiment or the like may be simplified or omitted, or a configuration which is not an essential configuration may be included in the pulse detector of the embodiment or the like.

As shown in Fig. 1, a pulsimeter which is an example of the electronic apparatus of the embodiment includes a pulse wave detection unit 10, a body motion detection unit 20, a pulse detector 100, and a display unit 70. However, the electronic apparatus is not limited to the configuration of Fig. 1, and various modifications may be made, in which some constituent elements are omitted or changed, other constituent elements are added, or the like.

The pulse wave detection unit 10 outputs a signal on the basis of sensor information (pulse wave sensor signal) of a pulse wave sensor. The pulse wave detection unit 10 can include, for example, a pulse wave sensor 11, a filter processing unit 15, and an A/D conversion unit 16. However, the pulse wave detection unit 10 is not limited to the configuration of Fig. 1, and various modifications may be made, in which some constituent elements are omitted, other constituent elements (for example, an amplification unit which amplifies a signal, and the like) are added, or the like.

The pulse wave sensor 11 is a sensor which detects a pulse wave signal, and for example, a photoelectric sensor or the like is considered. When a photoelectric sensor is used as the pulse wave sensor 11, a sensor which is configured to cut a signal component of external light, such as sunlight, may be used. This can be realized by, for example, providing a plurality of photodiodes and performing feedback processing or the like using the signals of the photodiodes to obtain difference information.

The pulse wave sensor 11 is not limited to a photoelectric sensor, and may be a sensor which uses an ultrasonic wave. In this case, the pulse wave sensor 11 has two piezoelectric elements, excites one piezoelectric element to transmit an ultrasonic wave into a living body, and receives a reflected wave of the ultrasonic wave by the blood flow of the living body by the other piezoelectric element. Since there is frequency change between the transmitted ultrasonic wave and the received ultrasonic wave due to the Doppler effect of the blood flow, in this case, it is possible to acquire a signal corresponding to the blood flow rate, and to estimate pulse information. As the pulse wave sensor 11, other sensors may be used.

The filter processing unit 15 performs high pass filter processing for the sensor information from the pulse wave sensor 11. Note that the cutoff frequency of the high pass filter may be obtained from a typical pulse rate. For example, there are very few cases where a usual pulse rate of a person falls below 30 per minute. That is, since there are very few cases where the frequency of a signal resulting from heartbeat is equal to or lower than 0.5 Hz, even if information of a frequency band in this range is cut, there ought to be little adverse effect on a signal desired to be acquired. Accordingly, the cutoff frequency may be set to about 0.5 Hz. In some cases, a different cutoff frequency, such as 1 Hz, may be set. Furthermore, since a typical upper limit value to the pulse rate of the person can be assumed, the filter processing unit 15 may perform band pass filter processing, instead of high pass filter processing. Although a cutoff frequency on a high frequency side may be freely set to some extent, for example, a value of 4 Hz or the like may be used.

The A/D conversion unit 16 performs A/D conversion processing and outputs a digital signal. The processing in the above-described filter processing unit 15 may be analog filter processing which is performed before the A/D conversion processing or digital filter processing which is performed after the A/D conversion processing.

The body motion detection unit 20 outputs a signal (body motion detection signal) according to body motion on the basis of the sensor information of various sensors. The body motion detection unit 20 can include, for example, an acceleration sensor 21, a pressure sensor 22, and an A/D conversion unit 26. However, the body motion detection unit 20 may include other sensors (for example, a gyro sensor), an amplification unit which amplifies a signal, or the like. It is not necessary to provide a plurality of types of sensors, and a configuration in which one type of sensor is included may be made.

The pulse detector 100 includes a signal processing unit 110 and a pulse information calculation unit 120. However, the pulse detector 100 is not limited to the configuration of Fig. 1, and various modifications may be made, in which some constituent elements are omitted, other constituent elements are added, or the like. The signal processing unit 110 performs signal processing for an output signal from the pulse wave detection unit or an output signal from the body motion detection unit.

The signal processing unit 110 can include a pulse wave signal processing unit 111 and a body motion signal processing unit 113.

The pulse wave signal processing unit 111 performs some kind of signal processing for a signal from the pulse wave detection unit 10. As the output from the pulse wave detection unit 10 represented by D1 of Fig. 1, various signals based on the pulse wave sensor signal are considered. For example, since there are many cases where the calculation of the pulse information described below is performed on the basis of the pulse wave sensor signal (pulse wave detection signal) after DC component cutting, it is assumed that the pulse wave sensor signal after the high pass filter processing is included in D1. However, a signal which is not subjected to filter processing may be output, or in some cases, a pulse wave sensor signal after low pass filter processing may be output. When a plurality of signals (for example, both a pulse wave sensor signal before the high pass filter processing and a pulse wave sensor signal after the processing) are included in D1, the processing in the pulse wave signal processing unit 111 may be performed for all signals included in D1 or may be performed for some signals. Various processing contents are considered, and for example, equalizer processing for the pulse wave detection signal may be performed, or other kinds of processing may be performed.

The body motion signal processing unit 113 performs various kinds of signal processing for the body motion detection signal from the body motion detection unit 20. Similarly to D1, as the output from the body motion detection unit 20 represented by D2, various signals are considered. In the example of Fig. 1, since the acceleration sensor 21 and the pressure sensor 22 are provided, the body motion detection signal of D2 includes an acceleration signal and a pressure signal. Since other sensors, such as a gyro sensor, may be used as a body motion detection sensor, output signals corresponding to the types of sensors are included in D2. The processing in the body motion signal processing unit 113 may be performed for all signals included in D2 or may be performed for some signals. For example, comparison processing of the signals included in D2 may be performed, thereby performing processing for determining a signal for use in noise reduction processing in a body motion noise reduction unit 122 described below.

Note that, in the processing in the pulse wave signal processing unit 111, the body motion detection signal may be used along with the signal from the pulse wave detection unit. Similarly, in the processing in the body motion signal processing unit 113, the signal from the pulse wave detection unit 10 may be used along with the body motion detection signal. A signal after required processing is performed for the output signal from the pulse wave detection unit 10 in the pulse wave signal processing unit 111 may be used for use in the processing in the body motion signal processing unit 113, or otherwise may be made.

The pulse information calculation unit 120 includes a body motion noise reduction unit 122, and calculates pulse information on the basis of the pulse wave detection signal (the pulse wave sensor signal after DC component cutting) after the body motion noise reduction processing.

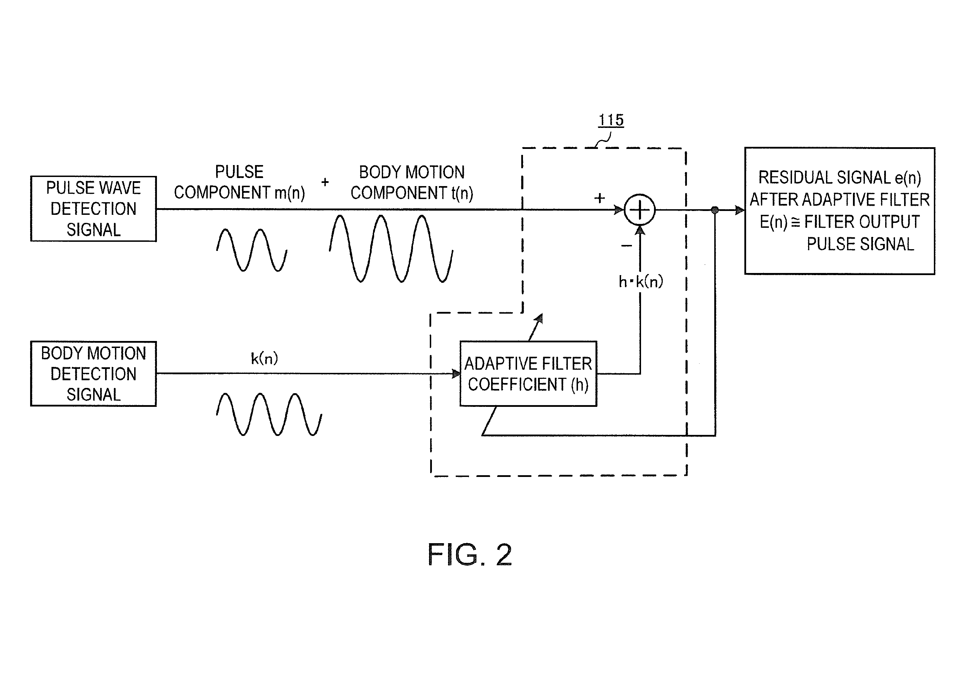

The body motion noise reduction unit 122 performs processing for reducing noise (body motion noise) due to body motion from the pulse wave detection signal using the body motion detection signal. A specific example of noise reduction processing using an adaptive filter is shown in Fig. 2. The pulse wave sensor signal acquired from the pulse wave sensor 11 includes a component due to body motion, in addition to a component due to heartbeat. The same applies to the pulse wave detection signal for use in the calculation of the pulse information. Of these, a component due to heartbeat is useful for the calculation of the pulse information, and the component due to body motion is obstructive to the calculation. Accordingly, a signal (body motion detection signal) due to body motion is acquired using a body motion sensor, and a signal component (called an estimated body motion noise component) correlated to the body motion detection signal is removed from the pulse wave detection signal, thereby reducing body motion noise included in the pulse wave detection signal. However, even if body motion noise in the pulse wave detection signal and the body motion detection signal from the body motion sensor are signals due to the same body motion, the signal levels are not identical. Accordingly, filter processing in which a filter coefficient is adaptively determined is performed for the body motion detection signal, whereby the estimated body motion noise component is calculated, and the difference between the pulse wave detection signal and the estimated body motion noise component is taken.

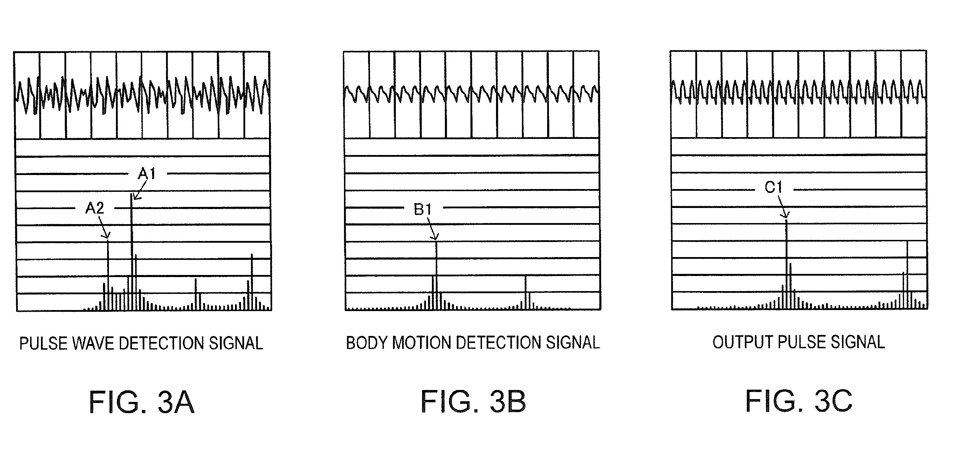

Figs. 3 (A) to 3 (C) illustrate the above-described processing by a frequency spectrum. In Fig. 3 (A) and the like, the time-varying waveform of a signal is shown on the upper side, and the frequency spectrum thereof is shown on the lower side. Fig. 3(A) shows a pulse wave detection signal before body motion noise reduction, and as indicated by A1 and A2, two frequencies having a large value appear in the spectrum. Of these, one frequency is due to heartbeat, and the other frequency is due to body motion. Although frequencies higher than A1 have a large value, since the frequencies are high-frequency components corresponding to an integer multiple of A1 and A2, in this case, these frequencies are not taken into consideration. Hereinafter, in Figs. 3(B) and 3(C), although high-frequency components are visible, similarly, in this case, these frequency components are not taken into consideration.

Meanwhile, Fig. 3(B) shows a body motion detection signal, and if one type of body motion is responsible for a body motion detection signal, as indicated by B1, a frequency having a large value appears. Here, the frequency of B1 corresponds to A2 of Fig. 3(A). In this case, the difference between the pulse wave detection signal and the estimated body motion noise component is taken by the method shown in Fig. 2, thereby obtaining a signal of Fig. 3 (C). As will be apparent from the drawing, the estimated body motion noise component having the peak B1 due to body motion is subtracted from the pulse wave detection signal having the two peaks A1 and A2 due to heartbeat and body motion, whereby a body motion component (corresponding to A2) in the pulse wave detection signal is excluded, and as a result, a peak C1 (whose frequency corresponds to A1) due to heartbeat remains.

When it is ensured that body motion noise included in the pulse wave detection signal corresponds to the body motion detection signal, no signal component which adversely affects the noise reduction processing is included in the body motion detection signal, or the like, since it is not necessary to perform frequency analysis in the body motion noise reduction unit 122, the frequency spectrum shown on the lower side of Figs. 3 (A) and 3 (B) may not taken into consideration. However, there is a case where the above-described condition is not satisfied depending on the type of a sensor which is used to acquire the body motion detection signal, or the like. In this case, for example, the body motion signal processing unit 113 may process the body motion detection signal such that the above-described condition is satisfied, or may exclude the body motion detection signal, which does not satisfy the above-described condition, from the output to the body motion noise reduction unit 122 or the like. Although various methods which determine whether or not the above-described condition is satisfied are considered, for example, the frequency spectrum which is obtained by frequency analysis and shown on the lower side of Figs. 3(A) and 3(B) may be used.

The pulse information calculation unit 120 calculates pulse information. The pulse information may be, for example, the value of a pulse rate. For example, the pulse information calculation unit 120 may perform frequency analysis, such as FFT, for the pulse wave detection signal after the noise reduction processing in the body motion noise reduction unit 122 to obtain a spectrum, and may perform processing for setting a representative frequency in the obtained spectrum as the frequency of heartbeat. In this case, a value which is a 60th power of the obtained frequency becomes a pulse rate (heart rate) which is generally used.

The pulse information is not limited to the pulse rate, and may be, for example, information (the frequency of heartbeat, the cardiac cycle, or the like) representing the pulse rate. The pulse information may be information representing the state of a pulse, or for example, a value representing a blood flow rate (or variation in blood flow rate) may be used as the pulse information. However, since there is an individual difference in the relationship between the blood flow rate and the signal value of the pulse wave sensor signal between users, it is preferable to perform correction processing for coping with the individual difference when the blood flow rate or the like may be used as the pulse information.

The timing at which a required value (an upper peak, a lower peak, a value equal to or greater than a required threshold value, or the like) appears on the time-varying waveform of the input pulse wave detection signal may be detected, and the cardiac cycle may be obtained from the time corresponding to the interval of the timing to calculate the pulse information. Alternatively, the waveform of the pulse wave detection signal may be deformed to a rectangular wave, and the rising edge of the rectangular wave or the like may be used to calculate the pulse information. In this case, since frequency analysis may not be performed, it is excellent in terms of the amount of calculation or power consumption. However, in this method, since the signal value is used as it is without performing conversion to the frequency axis, it is necessary to adjust the waveform to some extent, thus, it is preferable that frequency analysis is performed when there is much noise or the like.

The display unit 70 (in a broad sense, an output unit) displays various display screens which are used to present the calculated pulse information or the like, and can be realized by, for example, a liquid crystal display, an organic EL display, or the like.

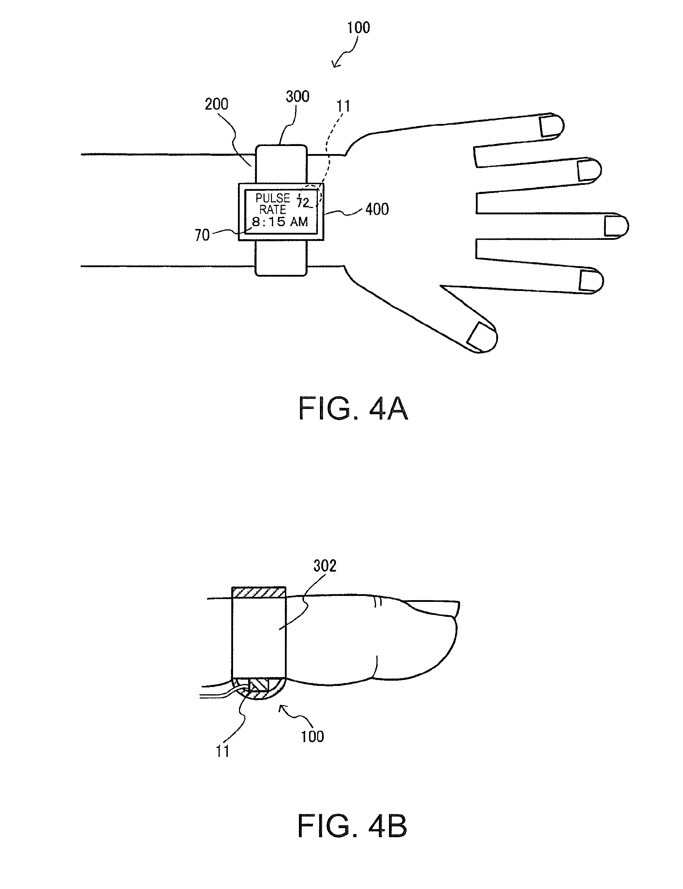

A specific example where the above-described electronic apparatus is a pulsimeter is shown in Figs. 4 (A) and 4 (B). Fig. 4 (A) shows an example of a wristwatch-type pulsimeter. A base portion 400 including the pulse wave sensor 11 and the display unit 70 is mounted on a left wrist 200 of the subject (user) by a holding mechanism 300 (for example, a band or the like) . Fig. 4 (B) shows an example of a finger-mounted type. The pulse wave sensor 11 is provided in the bottom portion of a ring-shaped guide 302 which is inserted into a fingertip of a subject. However, in the case of Fig. 4(B), since there is no space margin for providing the display unit 70, it is assumed that the display unit 70 (and a device corresponding to the pulse detector 100 as necessary) is provided in other ways.

Although a method of the embodiment described below can be applied to any type of electronic apparatus, it is more desirable that the method is applied to a wristwatch-type pulsimeter (the example of Fig. 4 (A)). However, in the example of Fig. 4 (A), the pulse wave sensor 11 is mounted in a region, such as the outside of the wrist (a region in contact with the rear cover surface of the wristwatch), in which the pulse wave sensor signal is difficult to acquire. For this reason, the amplitude of the pulse wave sensor signal output from the pulse wave sensor 11 generally tends to decrease. Accordingly, it is preferable to perform processing relating to precision of pulse information by any method.

2. Method of the embodiment

Next, the method of the embodiment will be described. As a recent electronic apparatus (in a narrow sense, a pulsimeter) including a pulse detector, a compact and lightweight device is also used. For example, in case of the wristwatch-type pulsimeter shown in Fig. 4(A), the user can walk, run, or perform other exercises even with the device mounted without any problem. From this, the situation in which the pulse detector is used is not limited to a rest state for medical purposes, and pulse information in an exercise state may be calculated and exercise load or the like may be notified to the user. In this case, since information of the number of steps, moving speed, or the like can be acquired using the above-described body motion sensor or the like as a pedometer, it is possible to present abundant information to the user.

From this background, a case where a method in which a pulse detector is mounted in a short span (for example, several minutes to several hours) only during diagnosis or during exercise and is also continuously mounted in a longer span (for example, half a day to several days) to acquire the log of biological information of the user is widely used is also considered.

However, in a constantly mounted device, since battery capacity is limited, power consumption becomes a major problem. Unless at least a continuous operation can be performed for half a day to several days even though charging is not made on the way, the above-described way to use is impossible.

In the method of the related art, a method which switches pulse information calculation processing on the basis of the presence/absence of a body motion detection signal is disclosed. Specifically, switching is made between a first method which performs frequency analysis by FFT and a second method which converts a pulse wave detection signal to a rectangular wave and uses information corresponding to the period (frequency) of the rectangular wave after conversion. As described above, since the second method has a smaller amount of calculation than the first method, the second method is appropriately used, thereby reducing power consumption. However, this method achieves reduction in the amount of calculation when there is little noise (that is, a certain level of precision can be secured even in the second method), and pulse information calculation is constantly performed. For this reason, it is necessary to constantly operate the pulse wave sensor 11 or the body motion sensor (acceleration sensor 21 or the like), and the effect of reducing power consumption is limited.

In this point, considering that a typical usage example of the pulse detector of the embodiment is the logging of the above-described biological information, it is considered that a pulse information calculation frequency can be decreased depending on the situation. That is, it should suffice that the pulse detector of the embodiment can detect the degree of variation when variation in pulse information is large, an abnormal value outside a normal state, or the like, and high-frequency and high-definition calculation is not required when variation in pulse information is small. This point is significantly different from when calculation with high precision is highly required constantly during measurement like the use for medical purposes.

Accordingly, the applicant proposes a method which determines the exercise state of the subject (user) and controls the operations of the respective units of the pulse detector (or the electronic apparatus including the pulse detector) on the basis of the determination result of the exercise state. Specifically, an exercise state corresponding to intensity of physical activity or the like is determined, and when intensive exercise is performed, it is considered that variation in pulse information is large and calculation processing is performed with high frequency (for example, constantly). On the other hand, the pulse information calculation frequency decreases during gentle exercise, and the pulse information calculation frequency further decreases during resting, sleeping, or the like. When decreasing the pulse information calculation frequency, since there are few advantages of performing sensing by the pulse wave sensor 11 or the body motion sensor with high frequency, the sensing operation rate of the sensor may be lowered corresponding to the pulse information calculation frequency.

Reduction in power consumption may be performed by controlling the contents of the noise reduction processing. For example, when performing multi-stage noise reduction processing (constituted by a multi-stage filter or the like having different characteristics), while a large number of (in a broad sense, all) filters are operated in case of intense exercise, the number of filters to be applied decreases as exercise is gentle, whereby reduction in power consumption is also achieved. The control of the embodiment based on the exercise state determination is control of at least one of the sensing operation rate of the pulse wave sensor 11, the sensing operation rate of the body motion sensor, the calculation processing rate of the pulse information, and the processing contents of the noise reduction processing, or a plurality of control targets may be controlled, and in this case, a combination thereof is arbitrary.

The intensity (intensity of physical activity) of exercise in the embodiment can be determined on the basis of various indexes. For example, determination may be made based on the amount of energy consumption by exercise using METs described in "Exercise Guide for Health Promotion 2006 - lifestyle-related disease prevention - (Exercise Guide 2006) " published by Ministry of Health, Labour and Welfare, or the like. In the embodiment, since control according to the degree of variation in pulse information is assumed, it is preferable to use an index in which exercise intensity per unit time is reflected, METs is appropriate rather than Ex (Exercise; the product of METs and the execution time) of Ministry of Health, Labour and Welfare or the like.

Here, a system configuration example of the pulse detector or the like will be described, and then a method of determining an exercise state will be described. Thereafter, a control method based on the determination result of the exercise state will be described, and finally, the details of processing of the embodiment will be described referring to a flowchart.

3. System configuration example

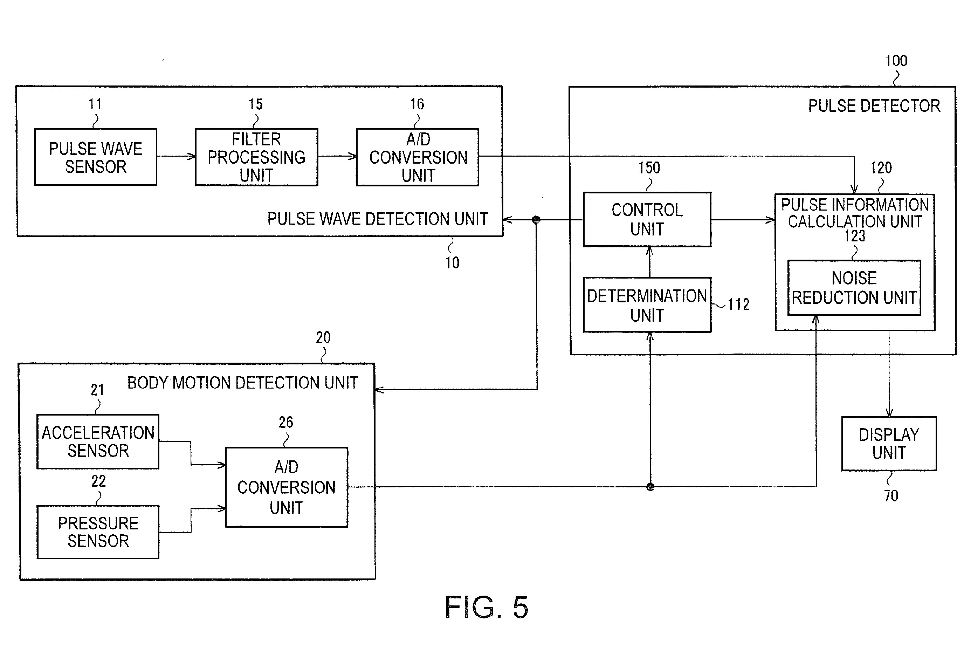

Fig. 5 shows a system configuration example of the electronic apparatus including the pulse detector of the embodiment. The electronic apparatus includes a pulse wave detection unit 10, a body motion detection unit 20, a pulse detector 100, and a display unit 70.

The pulse wave detection unit 10 includes a pulse wave sensor 11, a filter processing unit 15, and an A/D conversion unit 16. However, the pulse wave detection unit 10 is not limited to the configuration of Fig. 5, and various modifications may be made, in which some constituent elements are omitted, other constituent elements are added, or the like.

For the pulse wave sensor 11, as described above referring to Fig. 1, a photoelectric sensor or the like is used. In the embodiment, the filter processing unit 15 is realized by a high pass filter which performs high pass filter processing. The A/D conversion unit 16 converts an input analog signal to a digital signal and outputs the digital signal.

As shown in Fig. 5, the pulse wave sensor 11 is connected to the filter processing unit 15. The filter processing unit 15 is connected to the A/D conversion unit 16. The A/D conversion unit 16 is connected to the pulse information calculation unit 120, and outputs the pulse wave detection signal to the pulse information calculation unit 120. Various modifications to the connection of the respective units in the pulse wave detection unit 10 may be made.

The body motion detection unit 20 includes an acceleration sensor 21, a pressure sensor 22, and an A/D conversion unit 26. The acceleration sensor 21 and the pressure sensor 22 are connected to the A/D conversion unit 26. The A/D conversion unit 26 is connected to a determination unit 112 and a noise reduction unit 123 described below. The body motion sensor in the body motion detection unit 20 is not limited to that of Fig. 5, and a modification may be made, in which the pressure sensor 22 is omitted, other sensors are added, or the like.

The pulse detector 100 includes a determination unit 112, a pulse information calculation unit 120, and a control unit 150, and the pulse information calculation unit 120 includes a noise reduction unit 123. However, the pulse detector 100 is not limited to the configuration of Fig. 5, and various modifications may be made, in which some constituent elements are omitted, other constituent elements are added, or the like.

The determination unit 112 may be included in, for example, the signal processing unit 110 of Fig. 1, and determines the exercise state of the subject on which the pulse wave sensor 11 or the like is mounted. The determination of the exercise state is made on the basis of the body motion detection signal from the body motion detection unit 20 or the like, and the determination unit 112 outputs the determination result to the control unit 150. A method of determining the exercise state in the determination unit 112 will be described below.

As described referring to Fig. 1 or the like, the pulse information calculation unit 120 calculates the pulse information on the basis of the pulse wave detection signal. Specifically, noise reduction processing is performed by the noise reduction unit 123 for the pulse wave detection signal, and the pulse information is calculated using the pulse wave detection signal after the processing.

The noise reduction unit 123 performs the noise reduction processing for the pulse wave detection signal. Here, the noise reduction processing may include body motion noise reduction processing by the body motion noise reduction unit 122 shown in Fig. 2 or the like, or may be processing for reducing other kinds of noise other than body motion noise. When performing the body motion noise reduction processing, as described above, the processing is performed on the basis of the body motion detection signal from the body motion detection unit 20.

The control unit 150 performs control of the respective units on the basis of the determination result of the exercise state in the determination unit 112. Specifically, control of at least one of the sensing operation rate of the pulse wave detection unit 10 (in a narrow sense, the pulse wave sensor 11), the sensing operation rate of the body motion detection unit 20 (in a narrow sense, the body motion sensor), the calculation processing rate in the pulse information calculation unit 120, and the contents of the noise reduction processing in the noise reduction unit 123 is performed. The details of control will be described below.

As described referring to Fig. 1, the display unit 70 displays the pulse information which is the calculation result in the pulse information calculation unit 120. The determination result (current exercise state) in the determination unit 112 or the control contents (the sensing operation rate of the pulse wave detection unit 10 or the like) in the control unit 150 may be displayed as necessary.

4. Method of determining exercise state

Next, the method of determining the exercise state in the determination unit 112 will be described. Although various determination methods are considered, in this embodiment, it is assumed that the determination is made on the basis of the body motion detection signal from the body motion detection unit 20. Since it is assumed that the body motion detection signal is a signal based on the acceleration sensor 21, in the following description, although an acceleration detection value is used as the body motion detection signal, signals based on other body motion sensors may be used.

The magnitude of the acceleration detection value increases as exercise is intense. Accordingly, here, three exercise states of "exercising", "in living activity", and "sleeping" are considered in a descending order of exercise intensity, and the state of the subject is determined among the three states on the basis of the magnitude of the acceleration detection value. Note that the number of exercise states or the like may be set in various ways.

Since the higher the exercise intensity, the larger the magnitude the acceleration detection value, for example, two threshold values which satisfy $Th1 < Th2$ may be set, when the magnitude $A$ of the acceleration detection value satisfies $A < Th1$, it may be determined to be sleeping, when $Th1 \leq A < Th2$ is satisfied, it may be determined to be in living activity, and when $Th2 \leq A$ is satisfied, it may be determined to be exercising.

However, it is not desirable to determine the exercise state only from one acceleration detection value. For example, even when the user sits in a chair or lays on a bed and exercise intensity is low, if the user slightly moves the hand (in particular, the hand on which the acceleration sensor 21 is mounted) and collides against a desk, a wall, or the like, the magnitude of the acceleration detection value increases extremely. Considering the situation, it is not appropriate that it is determined to be exercising at this time, and it is necessary to suppress the effect of a value which is unexpectedly generated. Accordingly, in this embodiment, a required exercise state determination period is set, and determination is made on the basis of the acceleration detection value in this period. Although the length of the exercise state determination period can be freely set, for example, about one second may be used. In general, the operation period of the acceleration sensor 21 is considerably short (if the operation clock is 16 Hz, 1/16 seconds), and if the operation period is one second, a plurality of acceleration detection values (for example, 16 acceleration detection values) can be acquired.

Although various determination methods based on a plurality of acceleration detection values are considered, for example, an index value (for example, an average value, a median value, or the like) may be obtained from a plurality of acceleration detection values, and comparison processing of the index value and the above-described threshold values may be performed. Alternatively, comparison processing of each acceleration detection value and the above-described threshold values may be performed to create a histogram, and determination may be made on the basis of the histogram. With this, it is possible to suppress the possibility that an error occurs in the exercise state determination due to an abnormal value.

A difference in the possibility of state determination may be provided as necessary. For example, when pursuing reduction in power consumption, the possibility that it is determined to be sleeping may increase, and conversely, the possibility that it is determined to be exercising may decreases. If the time when it is determined that exercise intensity is low is extended, the effect of reducing power consumption increases.

When focusing on preventing missing of necessary biological information, the possibility that it is determined to be exercising may increases, and the possibility that it is determined to be sleeping may decrease. This is because, while actual exercise is intense, if it is determined that the state is in living activity or sleeping, the pulse information calculation or the noise reduction processing is omitted, pulse information (important information in the logging of the biological information) which intensively varies is acquired with low frequency (or with high frequency but with low precision).

That is, it is preferable to set whether it is readily determined that the exercise intensity of the subject who uses the pulse detector is high according to the usage, environment, or the like, or vice versa. Hereinafter, although three methods will be described as a specific method which provides a difference in the possibility of state determination, the processing may be realized by other methods.

In a first method, the setting of the above-described threshold values is changed. For example, if Th1 is large, since a numerical range in which it is determined to be sleeping is widened, as a result, it is readily determined to be sleeping. Similarly, if Th2 is small, since a numerical range in which it is determined to be exercising is widened, it is readily determined to be exercising.

In a second method, the determination based on the histogram is considered. When using the histogram, it is simply considered that a state having the maximum number of pieces of data among exercising, in living activity, and sleeping is determined as a final exercise state. Here, the determination conditions may be changed, and the number of pieces of data of a state in which a probability is desired to increase may not be maximal. For example, when the operation clock of the sensor is 16 Hz and the exercise state determination period is one second, 16 pieces of data are acquired in the single exercise state determination period. Accordingly, if the number of pieces of data in a certain state is not equal to or greater than six, the number of pieces of data in this state is unlikely to be maximal and is not maximal unless a larger or comparable value is taken depending on the number of pieces of data of a different state. However, if it will be readily determined to be sleeping, and if the number of pieces of data of sleeping is equal to or greater than five, it is appropriate to set a condition in which it is determined to be sleeping or the like without depending on a different number of pieces of data.

In a third method, the result in the single exercise state determination period is set as an intermediate result, instead of a final result, and a plurality of intermediate results are collected to obtain a final result. Specifically, processing is changed according to the relationship between a current state and a state after transition. In order that it is readily determined to be exercising, for example, when the current state is in living activity or sleeping, the state is transited to exercising (the final result that the state is exercising is output) immediately after an intermediate result indicating exercising is obtained even once. In order to allow transition from exercising to in living activity (sleeping), it is appropriate to set a condition in which an intermediate result indicating in living activity (sleeping) should be obtained continuously multiple times. With this, it is possible to increase the possibility of exercising in the final state transition without taking into consideration the difference in the probability of the determination in the single exercise state determination period.

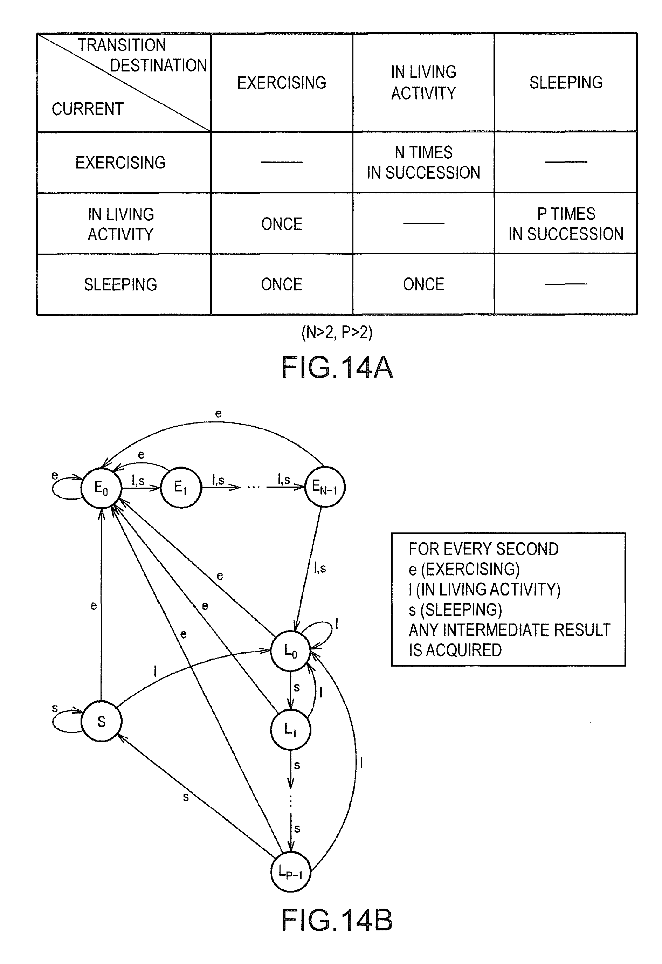

A specific example of the third method is shown in Figs. 14 (A) and 14 (B). Fig. 14 (A) corresponds to a method in which, as exercise intensity is high, the probability that it is determined to be in the state increases. In regard to transition from sleeping or in living activity to exercising, an intermediate result indicating exercising may be obtained even once. In regard to transition from sleeping to in living activity, since exercise intensity increases, a single intermediate result may be obtained.

In contrast, in regard to transition from exercising to in living activity, an intermediate result indicating in living activity (or sleeping) should be obtained continuously N (> 2) times. Similarly, in regard to transition from in living activity to sleeping, it is necessary that an intermediate result indicating sleeping is obtained continuously P (> 2) times. With this, while transition in an increasing direction of exercise intensity is easily performed, since transition in a decreasing direction of exercise intensity is difficult, as a result, it is possible to increase the possibility that it is determined to be an exercise state having high exercise intensity. This is shown in a state transition diagram of Fig. 14(B). The condition of state transition is not limited thereto, and for example, the number of times necessary for state transition may be changed, or when a plurality of intermediate results are required, change may be made, in which the intermediate results may not be continuous or the like.

In the above description, although the method based on the magnitude of the acceleration detection value has been described, the invention is not limited thereto. For example, the frequency may be obtained from the acceleration detection value, and the obtained frequency may be used for determination.

This method is effective for when periodic exercise, such as walking or running, is considered as a specific example of exercising. During walking or running, since exercise is repeated in a given rhythm, the acceleration detection value has periodicity. Motion in living activity has no periodicity since it is difficult to assume continuity in a given rhythm. That is, it is possible to distinguish between exercising and in living activity according to the presence/absence of periodicity. When determining sleeping, the magnitude of the above-described acceleration detection value may be used. That is, since the magnitude of the acceleration detection value is smaller than a required threshold value, it is determined to be sleeping, when the magnitude of the acceleration detection value is equal to or greater than the threshold value, determination of periodicity is made, if there is periodicity, it is determined to be exercising, and if there is no periodicity, it is determined to be in living activity.

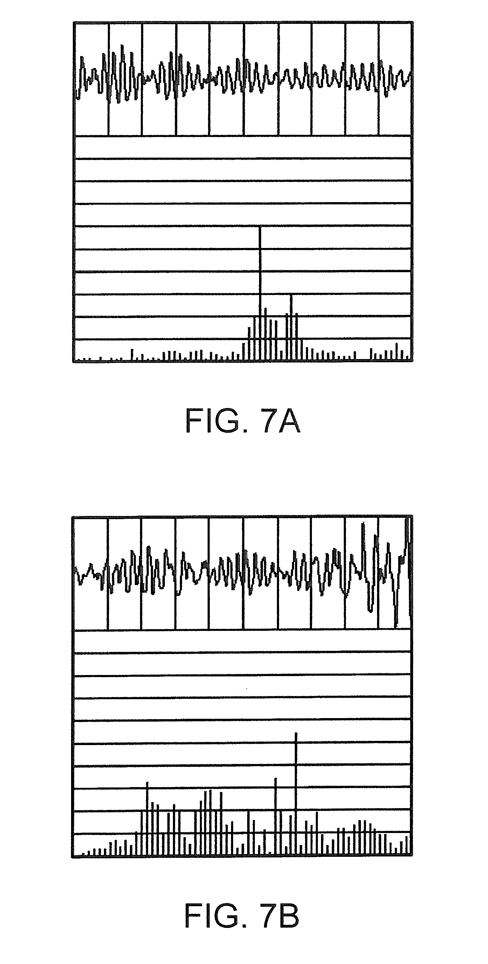

Although various ways of determination of the presence/absence of periodicity are considered, for example, frequency analysis, such as FFT, may be performed. A specific time-varying waveform during walking or running is shown on the upper side of Fig. 7 (A), and the frequency spectrum thereof is shown on the lower side of Fig. 7(A). A time-varying waveform corresponding to non-periodic motion is shown on the upper side of Fig. 7(B), and the frequency spectrum thereof is shown on the lower side of Fig. 7(B). As will be apparent from Figs. 7 (A) and 7 (B), when there is periodicity, there is a peak at the value of a required frequency in the frequency spectrum, and the value at a different frequency is considerably smaller than the peak. In contrast, when there is no periodicity, even if there is a peak at a required frequency, the ratio to the peak of the value of a different frequency increases. That is, the presence/absence of periodicity may be determined on the basis of the difference, and for example, comparison processing of the ratio of the peak value to a different value (for example, the fifth largest value or the like) and a required ratio threshold value or the like may be performed.

However, even when exercise, such as walking, which may be determined that there is periodicity is performed, since the walking pitch of the person is not constant strictly, and noise affects the sensor or the like, it is not always the case that the waveform of the body motion detection signal is a clean sine wave. For example, even when it should be determined that there is periodicity, as in the frequency spectrum of Fig. 7(A), a certain level of value appears at a frequency other than the peak. That is, "the presence/absence of periodicity" used herein is not a strict criterion on whether or not the body motion detection signal is a signal having a single frequency. On an assumption that a frequency other than a main frequency which is assumed to correspond to exercise is included, the presence/absence of frequency is determined according to how degree a frequency other than the main frequency is included. As the determination criterion, a required reference value may be used, or for example, the above-described ratio threshold value or the like may be used.

The periodicity of the body motion detection signal may be captured in terms of strength/weakness, instead of presence/absence. That is, as long as it is assumed that a frequency other than the main frequency is included, the strength/weakness of periodicity may be determined according to the degree of inclusion, and processing based on the determination result may be performed. The strength/weakness of periodicity may be represented by a required index value, and for example, the ratio of a different value (for example, the fifth largest value or the like) to the above-described peak value may be used. In this case, as periodicity is strong, the index value decreases, and as periodicity is weak, the index value increases. In the exercise state determination of the embodiment, when periodicity is strong, it is determined to be exercising, and when periodicity is weak, it is determined to be in living activity. When the presence/absence of periodicity is used, determination by two states of presence and absence is made. Meanwhile, when strength/weakness of periodicity is used, finer processing (for example, processing by three states of a state having strong periodicity, a state having intermediate periodicity, and a state having weak periodicity with two strength threshold values) may be performed.

5. Control method based on determination result of exercise state

Next, a method of performing control of the respective units of the electronic apparatus on the basis of the determination result of the exercise state will be described. Here, a method of controlling the sensing operation rate of the pulse wave detection unit 10 (in a narrow sense, the pulse wave sensor 11), the sensing operation rate of the body motion detection unit 20 (in a narrow sense, the body motion sensor), the calculation processing rate of the pulse information calculation unit 120, and the contents of the noise reduction processing in the noise reduction unit 123 will be described.

5.1 Control of pulse wave sensing operation

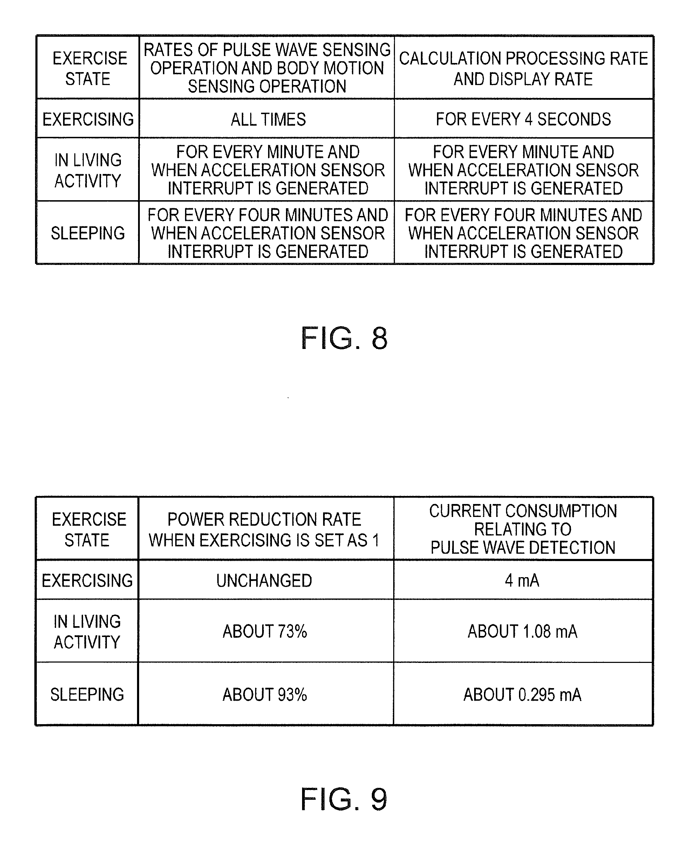

Fig. 8 shows an example of control according to the exercise state. For example, control is performed such that, while the pulse wave sensor 11 is constantly operated during exercising, the pulse wave sensor 11 is operated at every minute in living activity, is operated at every four minutes during sleeping, or the like. With this, since it is possible to shorten the operation time of the pulse wave sensor 11 as exercise intensity is low (that is, as it is assumed that variation in pulse information is small or the like), it becomes possible to reduce power consumption.

Even in living activity or during sleeping, the operation is not limited to once at every minute or at every four minutes, and as shown in Fig. 8, control may be performed such that the operation is made at the time of the occurrence of an acceleration sensor interrupt. In the example described referring to Fig. 14(B) or the like, it is assumed that the exercise state is transited to exercising when the acceleration sensor interrupt occurs. However, as will be easily understood considering turning over or the like during sleeping, while the actual exercise state is not changed, the acceleration detection value may increase. In this case, if the state of sleeping is kept instead of transiting the exercise state to exercising, it is efficient to determine whether or not the large value of the acceleration detection value continuously appears. Accordingly, even in living activity or during sleeping, the operation may be made at the time of the occurrence of the acceleration sensor interrupt.

However, in the following description, it is assumed that the operation at the time of the occurrence of the acceleration sensor interrupt in living activity or during sleeping is not taken into consideration. Even if the acceleration sensor interrupt is not taken into consideration, the condition of state transition among exercising, in living activity, and sleeping is set finely, thereby coping with the problems, such as turning over. For example, a state $E_S$ which represents exercising and is easy to transit to sleeping may be added in Fig. 14(B), and when transiting from sleeping to exercising, the transition to $E_S$ is first made. With this, even if the transition to exercising is made once due to turning over, the state can be returned to sleeping immediately.

The control (in a narrow sense, the control of the pulse wave sensor 11) of the pulse wave sensing operation of the embodiment is to change the operation rate, and the operation frequency (corresponding to the number of times in which a signal is acquired per second) may have, for example, a constant value of 16 Hz or the like. Here, the operation rate represents information corresponding to y / x when the operation is made for y seconds of a required time (x seconds) and stopped for the remaining x - y seconds. That is, power saving control of the embodiment is performed by increasing the time when the operation of the pulse wave sensor 11 is inactive, and it is not assumed that control for lowering the operation frequency from 16 Hz to 8 Hz or the like is performed.

This is because the resolution of the pulse information (pulse rate) to be calculated depends on the period of the pulse wave detection signal for use in FFT, instead of the operation frequency. For example, when FFT is performed using a signal for 16 seconds from the pulse wave sensor 11 which operates at 16 Hz, the resolution of the pulse rate is 3.75 beats/minute. If this level of resolution is provided, the pulse rate at one beat/minute can be estimated by interpolation processing or the like, and information can be presented finely to the user. Even if the operation frequency is set to 64 Hz, when a signal to be used is for 16 seconds, the resolution of the pulse rate is maintained at 3.75 beats/minute. When a signal for four seconds at the operation frequency of 64 Hz is used, even though a signal value for 256 samples same as 16 seconds at 16 Hz can be acquired, the resolution of the pulse rate falls to 15 beats/minute, making it difficult to present information in units of one beat.

That is, even if the operation frequency is improved, this does not contribute to the resolution of the pulse information and causes an increase in power consumption. Thus, in the pulse detector 100 of the embodiment, it is said that a high operation frequency is not required from the beginning. The operation frequency corresponds to the sampling frequency of FFT. For this reason, if the operation frequency increases, the Nyquist frequency increases, and a signal having a high frequency can be processed; however, even considering that the frequency of a signal due to heartbeat does not increase so much (for example, about 4 Hz at most), there is no need for increase the operation frequency. From the above, although a case where the control of the pulse wave sensor 11 is performed by the operation rate instead of the operation frequency has been described, the operation frequency may be controlled.

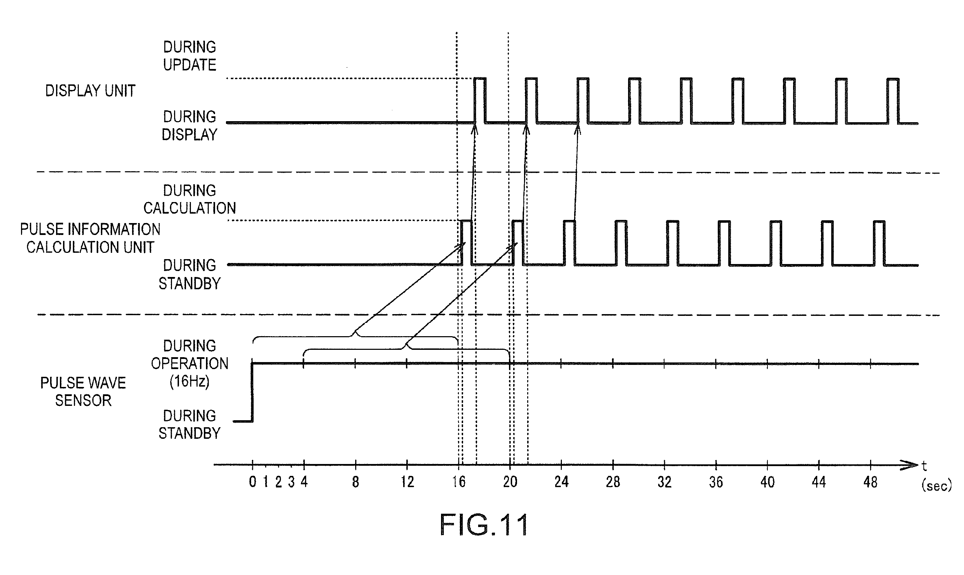

A specific example of the operation rate control is shown in Figs. 11 to 13. Fig. 11 shows the operation of the pulse wave sensor 11 or the like when it is determined to be exercising, and in this case, control for constantly operating the pulse wave sensor 11 is performed. With this, it is possible to suppress omission of acquisition of the pulse wave detection signal or the like when exercise intensity is high and variation in the pulse information is expected.

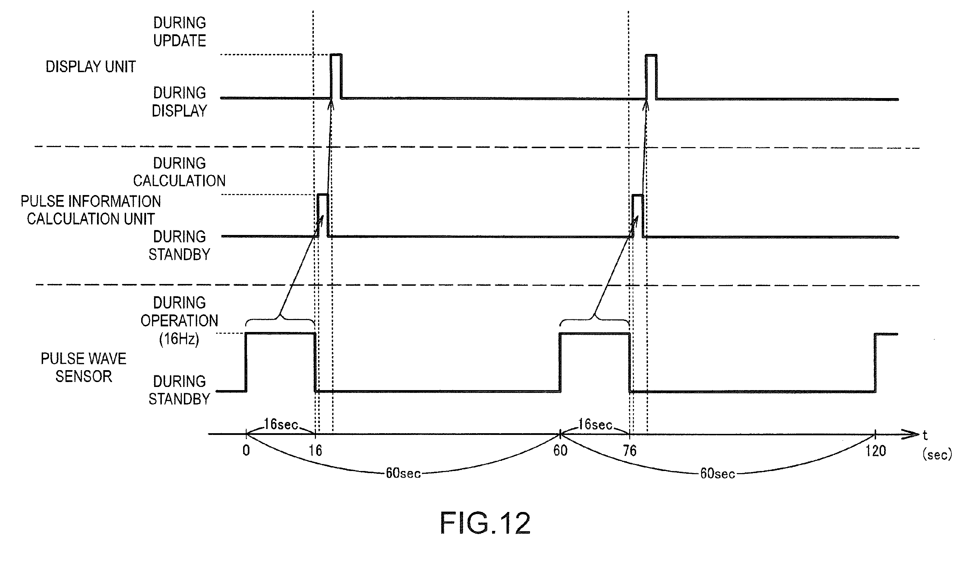

When it is determined to be in living activity, as shown in Fig. 12, the pulse wave sensor 11 is operated at every given time. As described above, the resolution of the pulse rate depends on the length of the FFT target period, and for example, it is necessary to set the period of about 16 seconds. A case where, if there is the time when the pulse wave sensor 11 is not operated (for example, the value of the pulse wave detection signal in this period becomes 0) in 16 seconds, the signal has discontinuity, or the like is considered, and this may adversely affect processing in FFT. Even if a signal value during a period in which the pulse wave sensor 11 is not operated is estimated by interpolation processing or the like, it is undeniable that precision in subsequent processing is degraded. That is, when operating the pulse wave sensor 11, it is desirable that continuous operation is made during a period enough not to adversely affect the processing in the pulse information calculation unit 120. For example, as shown in Fig. 12, when operating the pulse wave sensor 11, continuous operation may be made for 16 seconds in a required period (in Fig. 12, 60 seconds), and the remaining time may be set as a standby period. With this, since the operation of the pulse wave sensor 11 can be stopped for more time, it becomes possible to reduce power consumption.

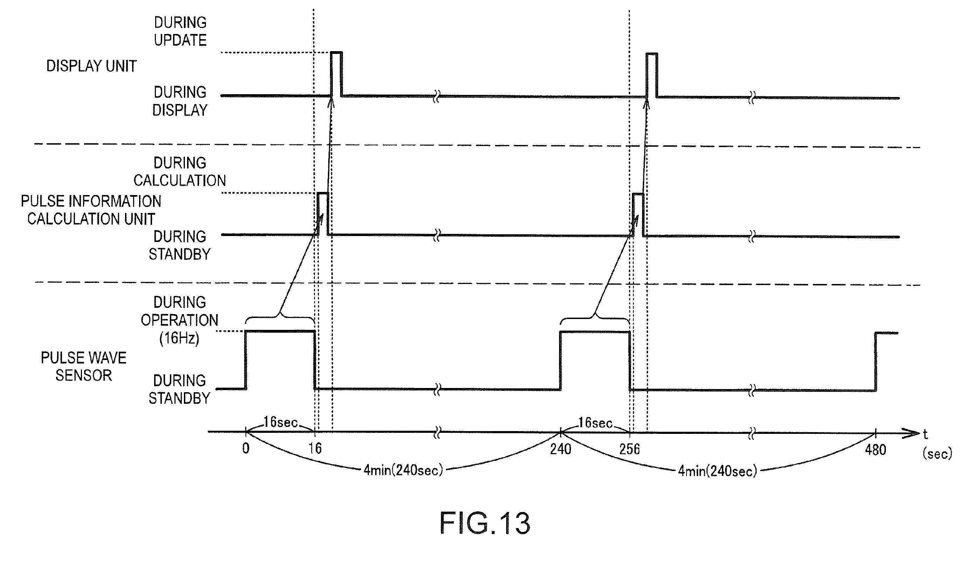

The control during sleeping is shown in Fig. 13. During sleeping, since it is considered that variation in pulse information or the like is unlikely to occur rather than in living activity, the standby period is set to be longer than in living activity. For example, as shown in Fig. 13, continuous operation may be made for 16 seconds in four minutes, and the remaining time may be set as a standby period. Similarly, it is desirable that a single operation period continues for about 16 seconds even during sleeping.

5.2 control of body motion sensing operation

Next, the control of the sensing operation in the body motion detection unit 20 will be described. Here, there is a problem in that a body motion sensor includes the above-described exercise state determination sensor which determines an exercise state and a body motion noise reduction sensor which is used for the body motion noise reduction processing in the noise reduction unit 123. Like the acceleration sensor 21, there is a sensor which can be used as an exercise state determination sensor and can also be used as a body motion noise reduction sensor. Hereinafter, an example of a method of controlling a body motion sensor based on a determination result of an exercise state will be described.

First, a body motion sensor which is assumed to be used as a body motion noise reduction sensor will be described. As described referring to Fig. 2 or the like, in regard to reduction in body motion noise, a body motion detection signal at the timing (in a narrow sense, the same timing) corresponding to the pulse wave detection signal to be subjected to the noise reduction processing is used. That is, in regard to the body motion sensor which is not used for processing other than the body motion noise reduction processing, even if a signal during a period in which the pulse wave detection signal is not acquired is acquired, since it is difficult to assume that the signal is used for the body motion noise reduction processing, unnecessary operation is made. Therefore, the body motion noise reduction sensor may perform the same control as in the pulse wave sensor 11.

Specifically, as shown in Figs. 11 to 13, continuous operation is made during exercising, and operation is made at a given interval in living activity or during sleeping. The interval is longer during sleeping than in living activity.

Next, a body motion sensor which is used as an exercise state determination sensor will be described. If the operation of the exercise state determination sensor is the same as the body motion noise reduction sensor (that is, the same as the pulse wave sensor 11 shown in Figs. 11 to 13), there is no particular problem during exercising; however, in living activity or during sleeping, the exercise state is not determined during the standby period (the period excluding 16 seconds in one minute or four minutes) of the sensor. That is, even if the exercise state of the subject changes during the standby period, this change cannot be detected until the next sensor operation period is reached. Accordingly, when there is change in a direction in which exercise intensity becomes more intense, such as transition from in living activity to exercising, while it is desirable to increase the pulse information calculation frequency and to capture change in pulse information finely, the control becomes difficult. Therefore, except in use environment in which change in the exercise state cannot be detected for a maximum one minute or four minutes, it is desirable that control is performed to allow the exercise state determination sensor to be operated at a high operation rate.

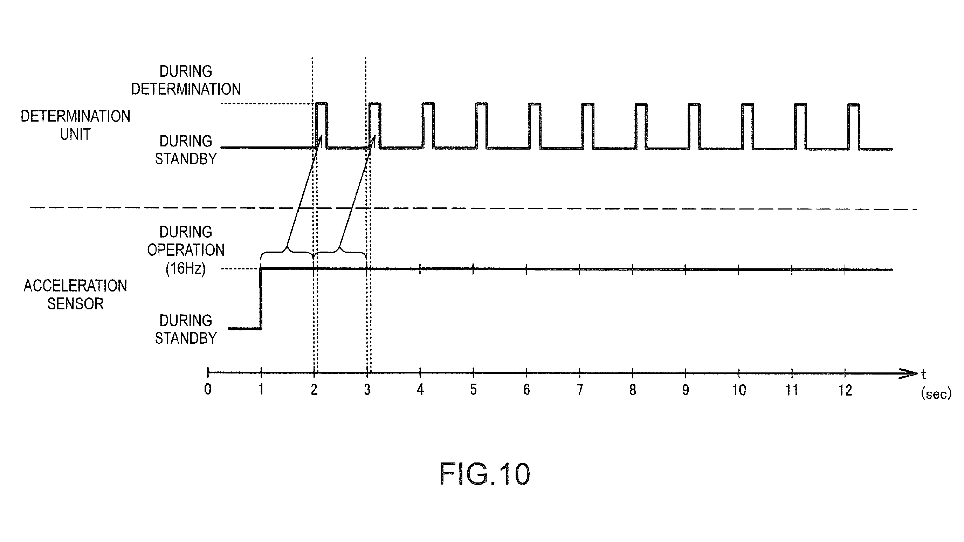

Here, although various methods are considered about how the operation rate of the exercise state determination sensor is set, for example, as shown in Fig. 10, continuous operation may be made. With this, as shown in Fig. 10, it is possible to perform determination at least once every second (at a higher frequency if a plurality of exercise state determination periods are set to overlap each other), and to follow change in the exercise state at high speed. However, the exercise state determination sensor is not limited to continuous operation, and a required standby period may be provided between adjacent operation periods. Here, as the standby period, a period is set such that, even if a delay corresponding to the length of the period occurs from when change in the actual exercise state occurs until the determination unit 112 detects the change, the delay does not affect subsequent processing. Since the determination of the exercise state is performed for a body motion detection signal in a required exercise state determination period (for example, one second), the length of the single operation period is set to a period equal to or longer than the exercise state determination period.

Like the acceleration sensor 21, a body motion sensor which is used as both an exercise state determination sensor and a body motion noise reduction sensor may be given priority to determine the exercise state and handled as an exercise state determination sensor.

5.3 Control of calculation processing rate of pulse information calculation unit

Next, the control of the calculation processing rate in the pulse information calculation unit 120 will be described. As described above, considering the resolution of the calculated pulse information (for example, the pulse rate), calculation processing using a pulse wave detection signal during a rather long (for example, about 16 seconds) period is required. However, as shown in Fig. 11, if a period suitable for a required pulse information calculation timing and a period suitable for a different pulse information calculation timing are allowed to overlap each other, it becomes possible to calculate the pulse information at a time interval shorter than the period. If a high calculation frequency is desired, calculation may be performed at each timing at which the pulse wave detection signal is acquired, and for example, if the operation frequency of the pulse wave sensor 11 is 16 Hz, the pulse information may be calculated every 1/16 seconds. However, if the calculation processing rate is too high, the amount of calculation and power consumption increase, and the pulse information which is presented to the user is frequently changed, making it difficult for the user to understand the pulse information. Accordingly, as shown in Fig. 11, the calculation of the pulse information may be performed at an interval of about four seconds, and when it is determined to be exercising in the determination unit 112, the calculation processing rate will be used.

When it is determined to be in living activity, since it is unlikely to be large variation in the pulse information, the calculation processing rate may be low. For example, as shown in Fig. 12, the pulse wave sensor 11 is operated only for 16 seconds in 60 seconds, and for this reason, when the pulse wave detection signal is acquired only for 16 seconds, the pulse information is calculated once every 60 seconds. This is because, even if a higher calculation processing rate is used, since no pulse wave detection signal is acquired during a part or the whole of the period subjected to FFT, there are doubts about the precision of the pulse information of the calculation result.

Similarly, when it is determined to be sleeping, the pulse information may be calculated once every 240 seconds.

Either the sensing operation rate of the pulse wave sensor 11 or the calculation processing rate of the pulse information calculation unit 120 may be controlled. Accordingly, control may be performed such that the pulse wave sensor 11 is constantly operated in all states of exercising, in living activity, and sleeping, and only the calculation processing rate is lowered. In this case, as shown in Fig. 12 or 13, since there is no case where the pulse information calculation timing is limited, the calculation processing rate can be set flexibly. However, considering that the embodiment achieves reduction in power consumption, it is necessary to lower the calculation processing rate in living activity to a significant level in terms of power saving compared to exercising, and to lower the calculation processing rate during sleeping to a significant level in terms of power saving compared to in living activity.

Conversely, control may be performed such that the pulse information calculation unit 120 is operated at a high rate (for example, once every four seconds) in all states of exercising, in living activity, and sleeping, and only the sensing operation rate of the pulse wave sensor 11 is lowered. However, in this case, since a situation in which no pulse wave detection signal is acquired during a part or the whole of the period subjected to FFT occurs frequently, as described above, a method which performs control while adjusting both the sensing operation rate and the calculation processing rate or controls the calculation processing rate without controlling the sensing operation rate is desirably used.

5.4 Control of contents of noise reduction processing of noise reduction unit

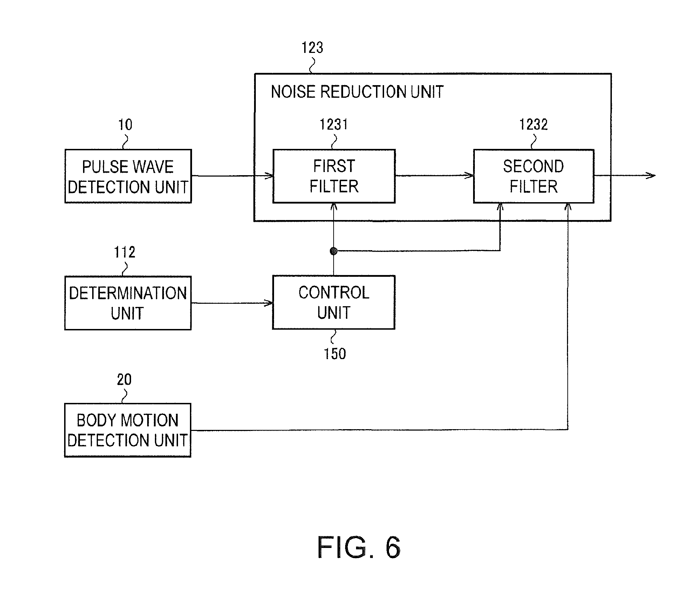

Next, the control of the noise reduction processing contents in the noise reduction unit 123 will be described. A configuration example of the noise reduction unit 123 is shown in Fig. 6. The noise reduction unit 123 includes a first filter 1231 and a second filter 1232. The first filter is an enhancement filter (for example, an adaptive spectral line enhancer or the like), and the second filter is a body motion noise reduction filter which is used for the body motion noise reduction processing described referring to Fig. 2 or the like. The first filter and the second filter are switched between active and inactive on the basis of a control signal from the control unit 150. In Fig. 6, although the pulse wave detection unit 10 and the first filter 1231 are connected together, the first filter 1231 and the second filter 1232 are connected together, and the pulse information is calculated on the basis of the output of the second filter 1232, a filter which is inactive by the control unit 150 may output an input signal without performing filter processing for the input signal. That is, the invention is not limited to a case where filter processing by all filters of the first filter and the second filter is performed, and filter processing by some filters may be performed and filter processing by other filters may not be performed. The entire filter processing may be skipped depending on the situation.

In the embodiment, the control of the contents of the noise reduction processing in the noise reduction unit 123 can be realized by control for switching the filters between active and inactive. If a number of filters are active, it can be expected that the noise reduction effect increases and subsequent pulse information calculation precision is improved. However, as the number of filters which are active increases, power consumption increases, thus, in this embodiment, as it is determined that exercise intensity is low, control is performed to decrease the number of filters which are active.

Although various specific methods are considered, for example, when it is determined to be exercising, both the first filter and the second filter are active and strong noise reduction processing is performed. When it is determined to be in living activity, the first filter is active, thereby removing noise which is suddenly mixed. Meanwhile, considering that body motion is small, the second filter is inactive and the body motion noise reduction processing is skipped. During sleeping, since it is unlikely to be large noise mixing, both the first filter and the second filter may be inactive.

A filter which is used in the noise reduction unit 123 is not limited to a two-stage filter. For example, if the body motion sensor includes a plurality of sensors, such as the acceleration sensor 21, the pressure sensor 22, and a gyro sensor, filters corresponding to the types of sensors may be used as the second filter. When the acceleration sensor 21 has a plurality of axes (for example, three axes), since filter processing based on the acceleration detection values of the respective axes may be performed, filters corresponding to the acceleration sensor 21 may be provided as many as the number of axes. In this way, a three- or more-stage filter can be provided, and in this case, a combination of filters which are active for the determination result of the exercise state or the like can be freely set. However, as described above, when exercise intensity is low, it is assumed that body motion noise is small, and the need for the body motion noise reduction processing is comparatively reduced. For this reason, in this reason, it is effective to make the filters included in the second filter inactive.

6. Details of processing

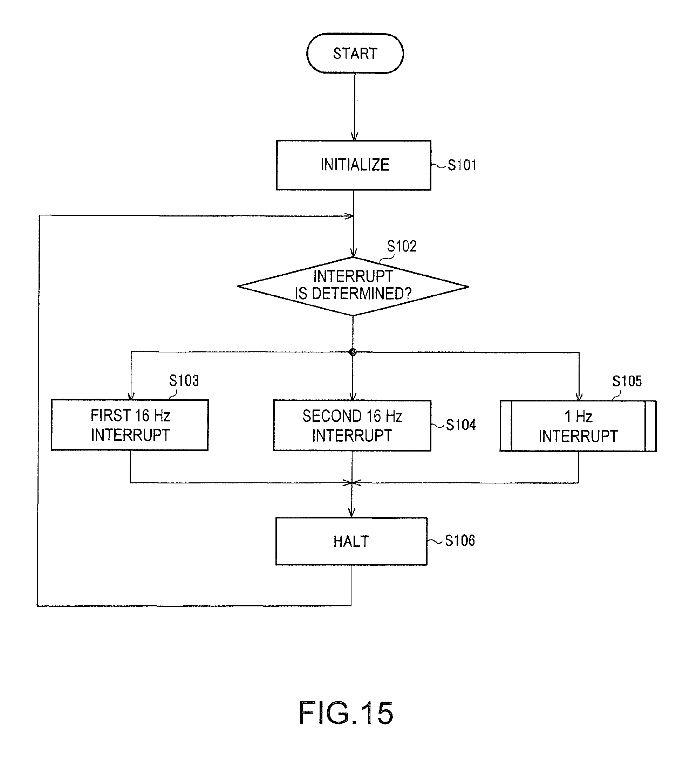

The details of the processing of the embodiment will be described referring to a flowchart or the like. Fig. 15 is a basic flowchart of the processing of the embodiment. If the processing starts, first, various kinds of initialization processing are performed (S101). Thereafter, interrupt waiting is made (S102), and when interrupt is performed, corresponding processing is performed. Specifically, processing of one of first 16 Hz interrupt (S103), second 16 Hz interrupt (S104), and 1 Hz interrupt (S105) is performed.

Here, the first 16 Hz interrupt is processing, such as sensing of the exercise state determination sensor (for example, the acceleration sensor 21) of the body motion sensor, and is performed at a time interval (for example, every 1/16 seconds) corresponding to the operation frequency of the sensor. The first 16 Hz interrupt may include A/D conversion processing for an acquired signal, noise reduction processing, or the like as well as sensing.

The second 16 Hz interrupt is processing, such as sensing of the pulse wave sensor 11 and the body motion noise reduction sensor (for example, the pressure sensor 22) of the body motion sensor, and is performed at a time interval (for example, every 1/16 seconds) corresponding to the operation frequency of the sensor. Similarly to the first 16 Hz interrupt, the processing may include A/D conversion processing or the like. However, as described above, since the second 16 Hz interrupt may be subjected to the operation rate control in the control unit 150, the second 16 Hz interrupt is not necessarily performed every 1/16 seconds, and the processing is likely to be inhibited during a required period.

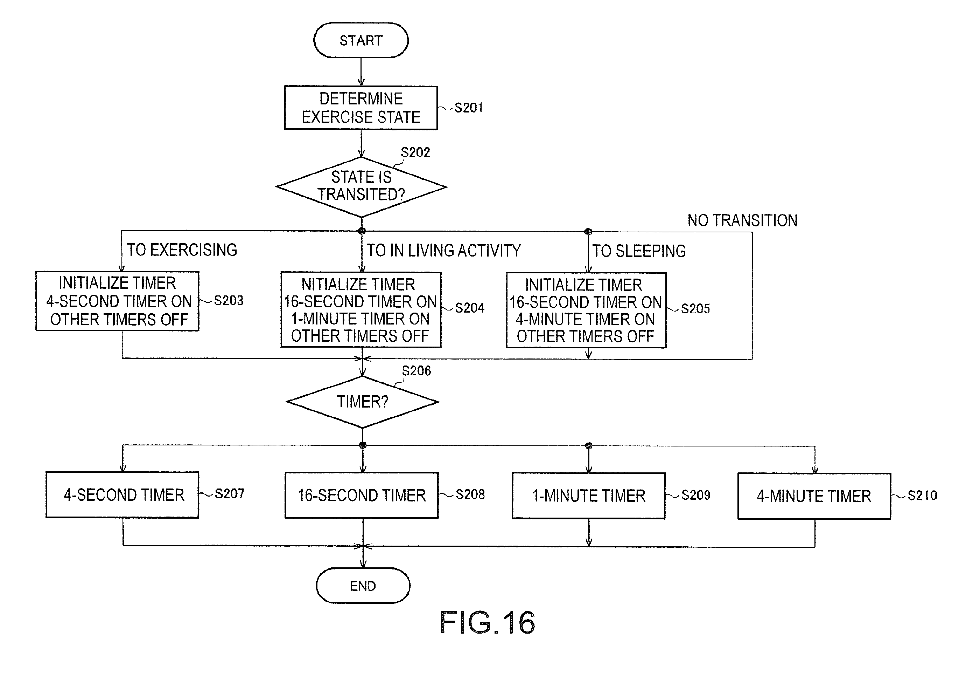

The 1 Hz interrupt is processing which is performed every second, and the details will be described below referring to Fig. 16. After the processing of S103 to S105, it waits until the next interrupt is generated (S106), and the process returns to S102.

Next, the details of the 1 Hz interrupt in S105 of Fig. 15 will be described referring to Fig. 16. If the processing stars, first, the determination of the exercise state is performed (S201). As shown in Fig. 10, for example, this is performed on the basis of the acceleration detection value in the previous one second. Various timers are set on the basis of the determination result of the exercise state. Specifically, it is determined whether or not state transition from a certain exercise state to a different exercise state is made (S202), and when the transition to exercising is made, all timers are initialized, then, a 4-second timer is active and other timers are inactive (S203). When the transition to in living activity is made, all timers are initialized, then, a 16-second timer and a 1-minute are active and other timers are inactive (S204). When the transition to sleeping is made, all timers are initialized, then, the 16-second timer and the 4-minute timer are active and other timers are inactive (S205). When the same exercise state continues, since timer information may be maintained, the processing is not particularly performed and the process returns to S206.

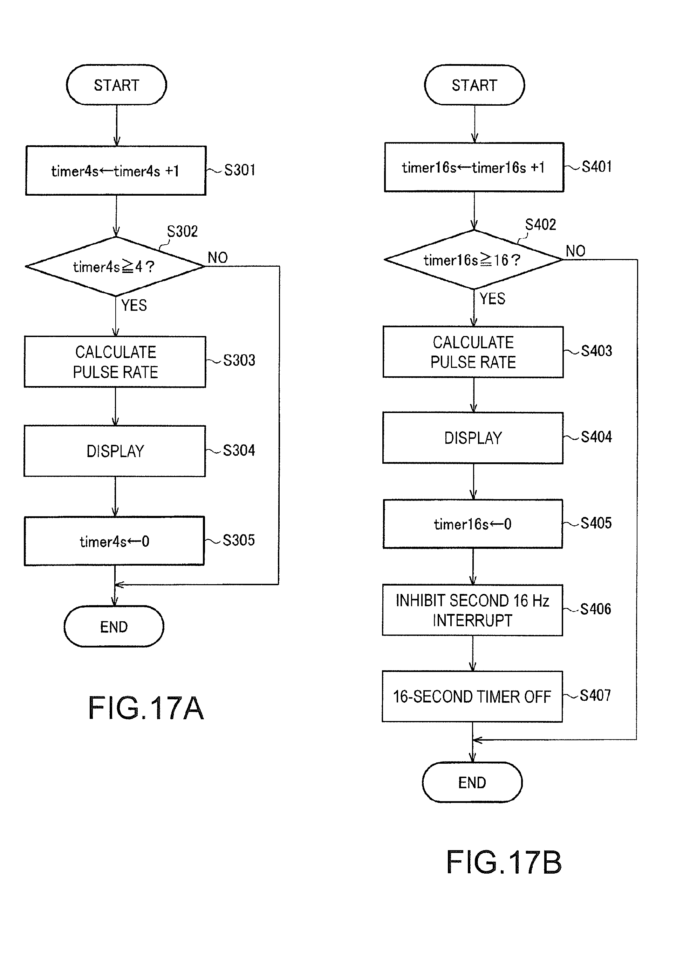

A timer which is active is determined (S206), and processing corresponding to the timer, which is active, is executed. Specifically, the processing of one of the 4-second timer (S207), the 16-second timer (S208), the 1-minute timer (S209), and the 4-minute timer (S210) is executed. In this example, the timers are not limited to as being exclusive operated, and a plurality of kinds of processing of S207 to S210 may be executed by the single processing of the flowchart of Fig. 16. For example, during a part of the period in living activity, the processing of both of the 16-second timer and the 1-minute timer is performed. Hereinafter, the details of the processing corresponding to the respective timers will be described referring to Figs. 17 (A) to 18 (B).

Fig. 17(A) is a flowchart illustrating the processing of the 4-second timer. The time of four seconds corresponds to the operation rate of the pulse information calculation unit 120 of Fig. 11, and the 4-second timer is a timer which is used when it is determined that the exercise state is exercising. If the processing starts, first, a variable (timer4s) which represents the lapse of time of the timer is incremented (S301), and it is determined whether or not the value is equal to or greater than four (S302). When it is determined in S302 to be Yes, since this corresponds to when four or more seconds (in a narrow sense, just four seconds) have elapsed from the previous processing (or at the time of the start of the system), the pulse information (pulse rate) calculation processing (S303) and the display of the calculation result (S304) are performed. For the next processing, the variable is initialized to 0 (S305), and the process ends. When it is determined in S302 to be No, since four seconds have not elapsed from the previous processing, the processing is not particularly performed and the process ends.

Fig. 17(B) is a flowchart illustrating the processing of the 16-second timer. The time of 16 seconds corresponds to the single operation period of the pulse wave sensor 11 of Figs. 12 and 13, and the 16-second timer is a timer which is used when it is determined that the exercise state is in living activity or sleeping. If the processing starts, first, a variable (timer16s) which represents the lapse of time of the timer is incremented (S401), and it is determined whether or not the value is equal to or greater than 16 (S402). When it is determined in S402 to be Yes, since this corresponds to the end timing of the operation period of the pulse wave sensor 11, the pulse information (pulse rate) calculation processing (S403) based on the pulse wave detection signal in the previous 16 seconds and the display of the calculation result (S404) are performed. For the next processing, the variable is initialized to 0 (S405). As will be understood from Figs. 12 and 13, subsequently, since the standby period of the pulse wave sensor 11 comes, the second 16 Hz interrupt corresponding to the pulse wave sensor 11 is inhibited (S406). At the same time, the 16-second timer is inactive (S407). When it is determined in S402 to be No, since 16 seconds of the operation period do not end, the processing is not particularly performed and the process ends. When the transition of the exercise state is not performed, the operation of the 16-second timer which is inactive in S407 is stopped until the 16-second timer is active by the 1-minute timer or the 4-minute timer described below.

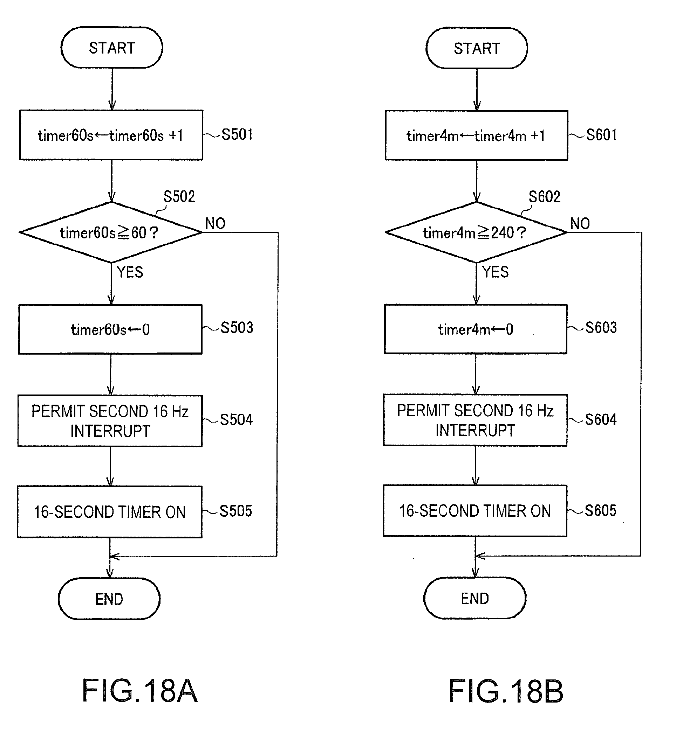

As shown in S204 and S205 of Fig. 16, in living activity, the 1-minute timer is active, and during sleeping, the 4-minute timer is active. Fig. 18(A) is a flowchart illustrating the processing of the 1-minute timer. The time of 1 minute corresponds to the total period of the single operation period of the pulse wave sensor 11 of Fig. 12 and the standby period, and the 1-minute timer is a timer which is used when it is determined that the exercise state is in living activity.

If the processing starts, first, a variable (timer60s) which represents the lapse of time of the timer is incremented (S501), and it is determined whether or not the value is equal to or greater than 60 (S502). When it is determined in S502 to be Yes, since this corresponds to the end timing of the standby period of the pulse wave sensor 11, the variable is initialized to 0 (S503), and the operation (second 16 Hz interrupt) of the pulse wave sensor 11 which is inhibited in S406 of Fig. 17(B) is permitted (S504). The 16-second timer for transition to the counting of the operation period is active (S505).

Fig. 18(B) is a flowchart illustrating the processing of the 4-minute timer. The time of 4 minutes corresponds to the total period of the single operation period of the pulse wave sensor 11 of Fig. 13 and the standby period, and the 4-minute timer is a timer which is used when it is determined that the exercise state is sleeping. The processing is the same as the processing of the 1-minute timer, thus detailed description will not be repeated.

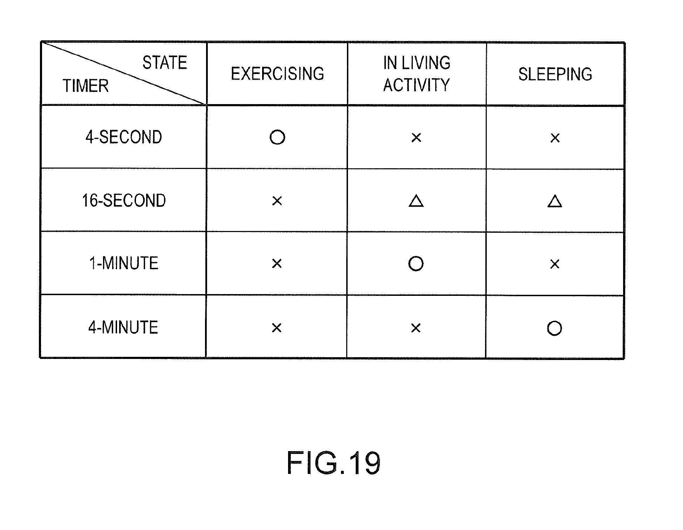

The relationship between each timer and the exercise state described above is shown in Fig. 19. However, a method of using each timer is not limited to the method of Fig. 19.

As shown in Fig. 19, during exercising, the four-second timer is used. As shown in Fig. 17(A), since there is no case where the second 16 Hz interrupt is inhibited in the 4-second timer, both the pulse wave sensor 11 and the body motion sensor (exercise state determination sensor and body motion noise reduction sensor) are constantly operated at 16 Hz. The 4-second timer processing is performed every second, and the pulse rate calculation and the display are performed once every four seconds. This corresponds to Fig. 11.

In living activity, two timers of the 16-second timer and the 1-minute timer are used. The exercise state determination sensor (acceleration sensor 21) is constantly operated. The pulse wave sensor 11 and the body motion noise reduction sensor (pressure sensor 22) are operated during the 16-second timer processing, the pulse rate calculation and the display are performed using the information at the time of the end of the operation period. After the 16-second timer detects the end (the lapse of 16 seconds) of the operation period, the operation (second 16 Hz interrupt) of the pulse wave sensor 11 and the body motion noise reduction sensor is inhibited, and the 16-second timer is inactive. The standby period comes until the 1-minute timer detects the lapse of 1 minute, and after the standby period ends, the second 16 Hz interrupt is permitted and then the 16-second timer is active, thereby restarting the operation period. Accordingly, insofar as the determination to be in living activity continues, the processing in which the 1-minute timer processing is performed every second and the 16-second timer is also operated every second for continuous 16 seconds in 60 seconds is repeated. This corresponds to Fig. 12.

During sleeping, processing in which the 1-minute timer in living activity is changed to the 4-minute timer is made, and the processing in which the 4-minute timer processing is performed every second and the 16-second timer is also operated every second for continuous 16 seconds in 4 minutes is repeated. This corresponds to Fig. 13.

In the embodiment, as shown in Fig. 5, the pulse detector 100 includes the pulse information calculation unit 120 which calculates the pulse information on the basis of the pulse wave detection signal from the pulse wave detection unit 10 having the pulse wave sensor 11 and the body motion detection signal from the body motion detection unit 20 having the body motion sensor (for example, the acceleration sensor 21), the determination unit 112 which determines the exercise state of the subject, and the control unit 150 which controls at least one of the pulse wave sensing operation in the pulse wave detection unit 10, the body motion sensing operation in the body motion detection unit 20, and the calculation processing rate in the pulse information calculation unit 120 on the basis of the determination result of the exercise state in the determination unit 112.

Here, the pulse wave detection signal is a signal which is primarily used for the pulse information calculation, and is, for example, an AC component (high-frequency component) of the pulse wave sensor signal which is the output of the pulse wave sensor 11. The body motion detection signal is a signal which is due to body motion, and includes a plurality of types of signals when the body motion detection unit 20 includes a plurality of body motion sensors (for example, both the acceleration sensor 21 and the pressure sensor 22).

Accordingly, it becomes possible to control the respective units of the electronic apparatus (in a narrow sense, a pulsimeter) including the pulse detector 100 on the basis of the determination result of the exercise state. Specifically, the sensing operation of the pulse wave detection unit 10 is controlled, and in this case, for example, the operation of the pulse wave sensor 11 may be controlled. Similarly, the operation of the body motion sensor, or the like is controlled, whereby the sensing operation of the body motion detection unit 20 may be controlled. The calculation processing rate of the pulse information calculation unit 120 may be controlled, and for example, control for changing the pulse information calculation interval is performed. Any one of the three types of control may be performed, two types may be performed in combination, or all the three types may be performed. With this control, it is possible to control the operation of the electronic apparatus according to the exercise state, making it possible to perform operation control according to the exercise state. Accordingly, it is possible to switch between a low power consumption state and a high power consumption state according to the exercise state, and to reduce overall power consumption, or the like.

Fig. 9 shows a reduction rate model of power consumption when the method of the embodiment is used. For example, if an operation current during the sensing operation is 4 mA and an operation current during the stopping of the sensing operation is 30 μA, as shown in Fig. 11, since continuous operation is made during exercising, an average operation current becomes 4 mA. In living activity, since the operation current is 4 mA for 16 seconds in 60 seconds

and 30 μA for the remaining 44 seconds, the average operation current becomes about 1.08 mA by Expression (1). Similarly, during sleeping, the average operation current becomes 0.295 mA by Expression (2).

$$4 \times (16 / 60) + 0.03 \times (44 / 60) = 1.089 \quad \cdots \quad (1)$$

$$4 \times (16 / 240) + 0.03 \times (224 / 240) = 0.295 \quad \cdots \quad (2)$$

The reduction rate of power consumption is obtained from the ratio of the average operation current, reduction of 73% can be made from Expression (3) in living activity, and reduction of 93% can be made from Expression (4) during sleeping.

$$100 - 1.089 / 4 \cong 73 \quad \cdots \quad (3)$$

$$100 - 0.295 / 4 \cong 93 \quad \cdots \quad (4)$$

The difference in battery life (continuous operation time) when the method of the related art and the method of the embodiment are used will be described. Here, it is assumed that the battery capacity is 70 mAh, and 24 hours are divided into 5 hours during exercising, 11 hours in living activity, and 8 hours during sleeping. Under this assumption, the daily average operation current in the method of the embodiment becomes 1.43 mA from Expression (5).

$$4 \times (5 / 24) + 1.089 \times (11 / 24) + 0.295 \times (8 / 24) = 1.43 \quad \cdots$$

$$(5)$$

That is, while battery life in the method of the related art becomes 17.5 hours from Expression (6), in the method of the embodiment, if the result of Expression (5) is used, continuous operation for about 49 times is possible from Expression (7).

$$70 / 4 = 17.5 \quad \cdots \quad (6)$$

$$70 / 1.43 \cong 49 \quad \cdots \quad (7)$$

The determination unit 112 may determine whether the subject is exercising or in living activity. When the determination unit 112 determines that the subject is in living activity, the control unit 150 performs at least one of control for lowering the operation rate of the pulse wave sensing operation, control for lowering the operation rate of the body motion sensing operation, and control for lowering the calculation processing rate compared to when it is determined that the subject is exercising.

The determination unit 112 may determine whether the subject is in living activity or sleeping. When the determination unit 112 determines that the subject is sleeping, the control unit 150 performs at least one of control for lowering the operation rate of the pulse wave sensing operation, control for lowering the operation rate of the body motion sensing operation, and control for lowering the calculation processing rate compared to when it is determined that the subject is in living activity.

The determination unit 112 may determine whether the subject is exercising or sleeping. When the determination unit 112 determines that the subject is sleeping, the control unit 150 performs at least one of control for lowering the operation rate of the pulse wave sensing operation, control for lowering the operation rate of the body motion sensing operation, and control for lowering the calculation processing rate compared to when it is determined that the subject is exercising.

Here, it is assumed that at least two of exercising, in living activity, and sleeping are considered as the exercise state of the subject, and the exercise intensity (for example, defined by the above-described METs or the like) has the relationship of exercising > in living activity > sleeping.

Accordingly, when it is determined to be in an exercise state having low exercise intensity, it is possible to perform the control for lowering at least one of the operation rate of the pulse wave sensing operation, the operation rate of the body motion sensing operation, and the calculation processing rate compared to when it is determined to be in an exercise state having high exercise intensity, thereby reducing power consumption, or the like. As power consumption can be reduced by the control, the operation rate or the calculation processing rate is lowered, whereby the acquisition rate of the pulse information, or the like is lowered. However, in the embodiment, since there is less need for pulse information calculation at a high rate with high precision when exercise intensity is low and intense change in pulse information is not assumed, it is considered that there is less adverse effect of the above-described control. As an example, as shown in Figs. 11 to 13, the pulse wave sensor 11 and the pulse information calculation unit 120 may be controlled, and in this case, the body motion sensor may perform the same operation rate control as in the pulse wave sensor 11.

The control unit 150 may control the contents of the calculation processing in the pulse information calculation unit 120 on the basis of the determination result of the exercise state in the determination unit 112.

Accordingly, it becomes possible to control the contents of the calculation processing in the pulse information calculation unit 120 in addition to the control of the operation rate or the calculation processing rate. In the embodiment, considering that it is typical to reduce power consumption when exercise intensity is low, it is considered that control is performed to change the contents of the calculation processing to a lower power consumption state (that is, a processing load is light) when exercise intensity is low compared to when exercise intensity is high.

As shown in Fig. 5, the pulse information calculation unit 120 may include the noise reduction unit 123 which performs the noise reduction processing for the pulse wave detection signal. The control unit 150 controls the contents of the noise reduction processing in the noise reduction unit 123 on the basis of the determination result of the exercise state.

Accordingly, as the control of the contents of the calculation processing in the pulse information calculation unit 120, specifically, it becomes possible to control the contents of the noise reduction processing in the noise reduction unit 123. During the processing in the pulse detector 100, considering that exercise (corresponding to body motion) becomes a major noise factor, it is assumed that the amount of noise is low in an exercise state having low exercise intensity compared to an exercise state having high exercise intensity. Therefore, when exercise intensity is low, even though a part of the noise reduction processing is omitted to achieve reduction in processing load (and reduction in power consumption), since it is considered that there is little adverse effect (degradation in precision of pulse information or the like) of the processing load, it becomes possible to perform efficient control in terms of power consumption or the like.

The determination unit 112 may determine whether the subject is exercising or in living activity. As shown in Fig. 6, the noise reduction unit 123 may have the first filter 1231 which performs enhancement filter processing, and the second filter 1232 which performs the noise reduction processing on the basis of the body motion detection signal. When it is determined that the subject is exercising, the noise reduction unit 123 performs the noise reduction processing by the first filter and the second filter. When it is determined that the subject is in living activity, the noise reduction processing is performed by the first filter. When it is determined to be in living activity, for example, the noise reduction processing may be performed using the first filter 1231 without using the second filter 1232.

Accordingly, it becomes possible to realize the contents of the noise reduction processing by switching the filters for use in the processing. As described above, in an exercise state having low exercise intensity, it is assumed that body motion noise due to body motion is low. Accordingly, as in Fig. 6, when the first filter 1231 performs the noise reduction processing which is not limited to body motion noise, and the second filter 1232 performs the noise reduction processing for body motion noise, since there is less need for the second filter 1232 due to low exercise intensity, it is efficient to preferentially omit the noise reduction processing in the second filter 1232.

The determination unit 112 may determine the exercise state on the basis of a body motion detection signal during a required exercise state determination period.

Accordingly, it becomes possible to determine the exercise state based on the body motion detection signal. Since it is assumed that the signal value of the body motion detection signal has a correspondence relationship with exercise intensity, it is possible to determine the exercise state based on the body motion detection signal. However, when the subject collides against other objects, or the like, even though actual exercise intensity is low, the signal value of the body motion detection signal increases instantaneously, thus, in the determination based on the body motion detection signal at this time, the exercise state is likely to be erroneously determined. Therefore, it is preferable to set a certain period and to perform processing using a plurality of body motion detection signals during the period, and in this case, the period is referred to as an exercise state determination period. Specifically, as shown in Fig. 10, about one second may be set, and if the operation frequency of the body motion sensor is 16 Hz, the body motion detection signals for 16 samples are acquired during the exercise state determination period.

The determination unit 112 may acquire intermediate results corresponding to one exercise state of exercising, in living activity, and sleeping on the basis of the body motion detection signals during the exercise state determination period and may determine the exercise state on the basis of one intermediate result or a plurality of intermediate results.

Accordingly, it becomes possible to not only determine the exercise state from the single determination result based on the body motion detection signals during the exercise state determination period and to determine the exercise state using a plurality of (in a narrow sense, a plurality of previous) determination results. With this, as shown in Fig. 14(A), it becomes possible to provide various conditions for the transition from a certain exercise state to a different exercise state, and to perform more efficient control or the like.

When it is determined that the subject is exercising, and when an intermediate result corresponding to an exercise state other than exercising is acquired N (where N is an integer equal to or greater than 2) times, the determination unit 112 may determine that the subject is in living activity. When it is determined that the subject is in living activity, and an intermediate result corresponding exercising is acquired once or M (where M is an integer which satisfies M < N and is equal to or greater than 2) times, it may be determined that the subject is exercising.

When it is determined that the subject is in living activity, and when an intermediate result corresponding to sleeping is acquired P (where P is an integer equal to or greater than 2) times, the determination unit 112 may determine that the subject is sleeping. When it is determined that the subject is sleeping, and when an intermediate result corresponding to in living activity is acquired once or Q (where Q is an integer which satisfies Q < P and is equal to or greater than 2) times, it may be determined that the subject is in living activity.

Accordingly, in regard to the transition of the exercise state, it becomes possible to set a condition in which the probability of transition to an exercise state having higher exercise intensity increase. Specifically, as shown in Fig. 14 (B), as the transition from exercising to in living activity or the transition from in living activity to sleeping, for transition in a direction in which exercise intensity is low, the determination based on a plurality of times (in a narrow sense, a plurality of continuous times; and the invention is not limited thereto) of intermediate results is required. Meanwhile, as the transition from in living activity to exercising or the transition from sleeping to in living activity, for transition in a direction in which exercise intensity is high, it should suffice that determination based on single intermediate result or a plurality of times (however, smaller than the number of times during the transition in a direction in which exercise intensity is lowered) of intermediate results is performed. When the actual exercise state of the subject is in a high exercise intensity state, it is assumed that the determination result in the determination unit 112 is a low exercise intensity state. Then, even though actual pulse information or the like is highly likely to vary intensively, and it is not possible to sufficiently cope with the variation due to degradation in the operation rate or the like. In this point, it is readily determined to be an exercise state having high exercise intensity, whereby it is possible to suppress the possibility of this situation and to suppress omission of acquisition of necessary information, or the like.

The determination unit 112 may count the frequency in which the signal value of the body motion detection signal exceeds a required signal threshold value during the exercise state determination period, and may determine that the subject is sleeping when the frequency is smaller than the required frequency threshold value.

Accordingly, it becomes possible to distinguish between sleeping and other exercise states on the basis of the magnitude of the signal value of the body motion detection signal. When the magnitude of the signal value is greater than a signal threshold value, since it can be inferred that exercise intensity is high, it is determined to be not sleeping. However, as described above, considering that a large signal value is acquired suddenly, when a value which exceeds the signal threshold value is acquired once, a state other than sleeping is highly likely to be erroneously determined, and in order to improve precision, it is desirable to perform comparison processing of the frequency in which the signal value exceeds the signal threshold value and a required frequency threshold value.

When it is determined that the subject is not sleeping, the determination unit 112 may perform determination about the presence/absence of periodicity of the body motion detection signal, and may determine that the subject is exercising when there is periodicity. When there is no periodicity, it may be determined that the subject is in living activity.

Accordingly, the invention is not limited to the determination by the signal value of the body motion detection signal, and determination based on periodicity is possible. This is effective when walking or running is assumed as an exercise state. In particular, if the presence/absence of periodicity is obtained by frequency analysis, such as FFT, it becomes possible to suppress the effect of noise or the like and to improve determination precision of the exercise state compared to a method in which the signal value of the body motion detection signal on the time axis is used as it is. Specifically, as shown in Figs. 7(A) and 7(B), the presence/absence of periodicity may be determined by comparison processing of a value at a frequency as a peak and a value at a different frequency, or the like. In case of sports, such as ball games, since there are many cases where body motion includes motion other than walking or running, even when exercise intensity is high, there may be no periodicity. When it is necessary to take this body motion into consideration, it is desirable to use a periodicity-independent method (or to use both a periodicity-independent method and a periodicity-based method).

As described above, periodicity may be captured by strength/weakness, instead of the presence/absence. That is, when it is determined that the subject is not sleeping, the determination unit 112 of the embodiment may perform determination about strength/weakness of periodicity of the body motion detection signal and may determine that the subject is exercising when it is determined that periodicity is strong. When it is determined that periodicity is weak, it may be determined that the subject is in living activity.

The determination unit 112 may determine whether the subject is exercising or in living activity. When the determination unit 112 determines that the subject is in living activity, the control unit 150 performs control for setting the operation rate of the pulse wave sensing operation to an operation rate whose ratio to the operation rate of the pulse wave sensing operation when it is determined that the subject is exercising is R1 (where R1 is a real number which satisfies $0 < R1 < 1$). The operation rate of the body motion sensing operation is set to an operation rate whose ratio to the operation rate of the body motion sensing operation when it is determined that the subject is exercising is R2 (where R2 is a real number which satisfies $0 < R1 < R2 \leq 1$).

Accordingly, when it is determined that exercise intensity is low (in living activity), it is possible to differ the degree of lowering of the operation rate based on a high exercise intensity state (exercising) between the pulse wave sensing operation and the body motion sensing operation. Referring to Figs. 11 and 12, while, in Fig. 11, the pulse wave sensor 11 is operated during the entire period (operation rate = 1), in Fig. 12, 16 seconds in 60 seconds become an operation period and the remaining 44 seconds become a standby period (operation rate = 16 / 60). In this case, R1 becomes 16 / 60. Here, there is a problem in that the body motion sensor is used for the determination of the exercise state. That is, when performing the same operation rate control as in the pulse wave sensor 11, in the example of Fig. 12, it is not possible to determine the exercise state for 44 seconds of the standby period. In this case, even though the exercise state during the standby period is changed (in particular, changed in a direction in which exercise intensity is high), since the determination in the determination unit 112 is not performed, it is not preferable in that the operation of the pulse wave sensor 11 continues to be stopped. From this, even though exercise intensity is low, the operation rate of the body motion sensing operation is not lowered as much as the operation rate of the pulse wave sensing operation, and the ratio R2 may satisfy $R2 > R1$. In a narrow sense, $R2 = 1$, and in this case, even though exercise intensity is low, the body motion sensor operates at the same operation rate as the high exercise intensity state. Specifically, as in Fig. 10, continuous operation may be made.

The body motion detection unit 20 may have an exercise state determination sensor and a body motion noise reduction sensor as a body motion sensor. The control unit 150 controls the sensing operation of the body motion noise reduction sensor on the basis of the determination result of the exercise state in the determination unit 112.

Here, the exercise state determination sensor means that a signal from the sensor is used for the determination of the exercise state. On the other hand, the body motion noise reduction sensor means that a signal from the sensor is used for the body motion noise reduction processing in the noise reduction unit 123. Like the acceleration sensor 21, although there is a sensor which is configured such that a signal from the sensor is used for both the determination of the exercise state and the body motion noise reduction processing, this body motion sensor may be included in the exercise state determination sensor.

Accordingly, in regard to the control based on the determination result of the exercise state, it becomes possible to include the body motion noise reduction sensor in the control targets. As described above, although the exercise state determination sensor should lower the operation rate to take into consideration the effect of delay in following variation in the actual exercise state, the body motion noise reduction sensor is not the case. If the target of the noise reduction processing using the body motion detection signal from the body motion noise reduction sensor becomes the pulse wave detection signal during a required period for the pulse information calculation in the pulse information calculation unit 120, it should suffice that the body motion noise reduction sensor can acquire the body motion detection signal during a period (in a narrow sense, the same period) corresponding to the required period. Therefore, even if the body motion noise reduction sensor is included in the target of the operation rate control, there are few problems.

The control unit 150 may perform control for excluding the sensing operation of the exercise state determination sensor from the control targets based on the determination result of the exercise state in the determination unit 112.

Accordingly, it becomes possible to set the operation rate of the exercise state determination sensor without depending on the exercise state. Therefore, the operation is made at an operation rate enough to follow variation in the actual exercise state regardless of the exercise state, whereby it becomes possible to suppress omission of calculation of necessary pulse information (or omission of acquisition of the pulse wave detection signal at a necessary timing) or the like.

The above-described embodiment can be applied to a pulse detector including the pulse information calculation unit 120 which calculates the pulse information on the basis of the pulse wave detection signal from the pulse wave detection unit 10 having the pulse wave sensor 11 and the body motion detection signal from the body motion detection unit 20 having the body motion sensor, the determination unit 112 which determines the exercise state of the subject, and the control unit 150 which performs control on the basis of the determination result of the exercise state in the determination unit 112. The pulse information calculation unit 120 includes the noise reduction unit 123 which performs the noise reduction processing for the pulse wave detection signal. The control unit 150 controls the contents of the noise reduction processing in the noise reduction unit on the basis of the determination result of the exercise state.

Accordingly, it becomes possible to control the contents of the noise reduction processing in the noise reduction unit 123 according to the exercise state separately from the operation rate of the sensing operation, the calculation processing rate, or the like. Specifically, as described above, each filter in the multi-stage filter may be switched between active and inactive modes, or the contents of the noise reduction processing may be changed by other methods.

The above-described embodiment can be applied to an electronic apparatus including the pulse detector 100, the pulse wave detection unit 10 and the body motion detection unit 20.

Accordingly, the method of the embodiment can be applied to an electronic apparatus including the pulse detector. The electronic apparatus is specifically a pulsimeter, and the configuration thereof may be as shown in Fig. 4(A), or a different configuration may be made.

In the pulse detector 100 of the embodiment, a part or a most part of the processing may be realized by a program. In this case, a processor, such as a CPU, executes the program, whereby the pulse detector 100 of the embodiment is realized. Specifically, the program stored in an information storage medium is read, and the read program is executed by the processor, such as a CPU. Here, the information storage medium (computer-readable medium) stores a program, data, and the like, and the function thereof can be realized by an optical disc (DVD, CD, or the like), an HDD (hard disk drive), a memory (card-type memory, ROM, or the like), or the like. The processor, such as a CPU, performs various kinds of processing of the embodiment on the basis of the program (data) stored in the information storage medium. That is, the information storage medium stores a program which causes a computer (a device including an operating unit, a processing unit, a storage unit, and an output unit) to function as the respective units of the embodiment (a program which causes a computer to execute the processing of the respective units).

As described above, although the embodiment has been described in detail, it can be easily understood by those skilled in the art that many modifications may be made without departing from the new matter and effects of the invention. Accordingly, all the modification examples fall within the scope of the invention. For example, in the specification and the drawings, there are some terms which are presented at least once together with other terms having a broader meaning or the same meaning. Each of the terms can be replaced with the corresponding other term at any location in the specification and the drawings. All combinations of the embodiment and the modification examples fall within the scope of the invention. The configuration and operation of the pulse detector and the electronic apparatus are not limited to those described in the embodiment, and various modifications may be made.

Reference Signs List

10: pulse wave detection unit, 11: pulse wave sensor, 15: filter processing unit, 16: A/D conversion unit, 20: body motion detection unit, 21: acceleration sensor, 22: pressure sensor, 26: A/D conversion unit, 70: display unit, 100: pulse detector, 110: signal processing unit, 111: pulse wave signal processing unit, 112: determination unit, 113: body motion signal processing unit, 120: pulse information calculation unit, 122: body motion noise reduction unit, 123: noise reduction unit, 150: control unit, 300: holding mechanism, 302: guide, 400: base portion, 1231: first filter, 1232: second filter.

**Claims**

1. A pulse detector comprising:

   a pulse information calculation unit which calculates pulse information on the basis of a pulse wave detection signal from a pulse wave detection unit having a pulse wave sensor and a body motion detection signal from a body motion detection unit having a body motion sensor;
   a determination unit which determines the exercise state of a subject; and
   a control unit which controls at least one of a pulse wave sensing operation in the pulse wave detection unit, a body motion sensing operation in the body motion detection unit, and a calculation processing rate in the pulse information calculation unit on the basis of the determination result of the exercise state in the determination unit.

2. The pulse detector according to claim 1,
   wherein the determination unit determines whether the subject is exercising or in living activity, and
   the control unit performs at least one of control for lowering the operation rate of the pulse wave sensing operation, control for lowering the operation rate of the body motion sensing operation, and control for lowering the calculation processing rate when the determination unit determines that the subject is in living activity compared to when it is determined that the subject is exercising.

3. The pulse detector according to claim 1,
wherein the determination unit determines whether the subject is in living activity or sleeping, and
the control unit performs at least one of control for lowering the operation rate of the pulse wave sensing operation, control for lowering the operation rate of the body motion sensing operation, and control for lowering the calculation processing rate when the determination unit determines that the subject is sleeping compared to when it is determined that the subject is in living activity.

4. The pulse detector according to claim 1,
wherein the determination unit determines whether the subject is exercising or sleeping, and
the control unit performs at least one of control for lowering the operation rate of the pulse wave sensing operation, control for lowering the operation rate of the body motion sensing operation, and control for lowering the calculation processing rate when the determination unit determines that the subject is sleeping compared to when it is determined that the subject is exercising.

5. The pulse detector according to any one of claims 1 to 4,
wherein the control unit controls calculation processing contents in the pulse information calculation unit on the basis of the determination result of the exercise state in the determination unit.

6. The pulse detector according to claim 5,
wherein the pulse information calculation unit includes a noise reduction unit which performs noise reduction processing for the pulse wave detection signal, and
the control unit controls the contents of the noise reduction processing in the noise reduction unit on the basis of the determination result of the exercise state.

7. The pulse detector according to claim 6,
wherein the determination unit determines whether the subject is exercising or in living activity, and
the noise reduction unit has a first filter which performs enhancement filter processing, and a second filter which performs the noise reduction processing on the basis of the body motion detection signal, performs the noise reduction processing by the first filter and the second filter when it is determined that the subject is exercising, and performs the noise reduction processing by the first filter when it is determined that the subject is in living activity.

8. The pulse detector according to any one of claims 1 to 7,
wherein the determination unit performs the determination of the exercise state on the basis of the body motion detection signal in a required exercise state determination period.

9. The pulse detector according to claim 8,
wherein the determination unit acquires an intermediate result corresponding to the exercise state of one of exercising, in living activity, and sleeping on the basis of the body motion detection signal in the exercise state determination period and determines the exercise state on the basis of one or a plurality of intermediate results.

10. The pulse detector according to claim 9,
wherein, when it is determined that the subject is exercising, the determination unit determines that the subject is in living activity when the intermediate result corresponding to the exercise state other than exercising is acquired N (where N is an integer equal to or greater than 2) times, and
when it is determined that the subject is in living activity, the determination unit determines that the subject is exercising when the intermediate result corresponding to exercising is acquired once or M (where M is an integer which satisfies M < N and is equal to or greater than 2) times.

11. The pulse detector according to claim 9 or 10,
wherein, when it is determined that the subject is in living activity, the determination unit determines that the subject is sleeping when the intermediate result corresponding to sleeping is acquired P (where P is an integer equal to or greater than 2) times, and
when it is determined that the subject is sleeping, the determination unit determines that the subject is in living activity when the intermediate result corresponding to in living activity is acquired once or Q (where Q is an integer which satisfies Q < P and is equal to or greater than 2) times.

**12.** The pulse detector according to claim 8,
wherein the determination unit counts a frequency in which the signal value of the body motion detection signal exceeds a required signal threshold value in the exercise state determination period and determines that the subject is sleeping when the frequency is smaller than a required frequency threshold value.

**13.** The pulse detector according to claim 12,
wherein, when it is determined that the subject is not sleeping, the determination unit performs determination on the presence/absence of periodicity of the body motion detection signal, determines that the subject is exercising when there is periodicity, and determines that the subject is in living activity when there is no periodicity.

**14.** The pulse detector according to claim 1,
wherein the determination unit determines whether the subject is exercising or in living activity, and
when the determination unit determines that the subject is in living activity,
the control unit performs control for setting the operation rate of the pulse wave sensing operation to the operation rate whose ratio to the operation rate of the pulse wave sensing operation when it is determined that the subject is exercising is R1 (where R1 is a real number which satisfies $0 < R1 < 1$), and
the control unit performs control for setting the operation rate of the body motion sensing operation to the operation rate whose ratio to the operation rate of the body motion sensing operation when it is determined that the subject is exercising is R2 (where R2 is a real number which satisfies $0 < R1 < R2 \leq 1$).

**15.** The pulse detector according to any one of claims 1 to 14,
wherein the body motion detection unit has an exercise state determination sensor and a body motion noise reduction sensor as the body motion sensor, and
the control unit controls the sensing operation of the body motion noise reduction sensor on the basis of the determination result of the exercise state in the determination unit.

**16.** The pulse detector according to claim 15,
wherein the control unit performs control for excluding the sensing operation of the exercise state determination sensor from a control target based on the determination result of the exercise state in the determination unit.

**17.** A pulse detector comprising:

a pulse information calculation unit which calculates pulse information on the basis of a pulse wave detection signal from a pulse wave detection unit having a pulse wave sensor and a body motion detection signal from a body motion detection unit having a body motion sensor;
a determination unit which determines the exercise state of a subject; and
a control unit which performs control on the basis of the determination result of the exercise state in the determination unit,
wherein the pulse information calculation unit includes a noise reduction unit which performs noise reduction processing for the pulse wave detection signal, and
the control unit controls the contents of the noise reduction processing in the noise reduction unit on the basis of the determination result of the exercise state.

**18.** An electronic apparatus comprising:

the pulse detector according to any one of claims 1 to 17;
the pulse wave detection unit; and
the body motion detection unit.

**19.** A program which causes a computer to function as:

a pulse information calculation unit which calculates pulse information on the basis of a pulse wave detection signal from a pulse wave detection unit having a pulse wave sensor and a body motion detection signal from a body motion detection unit having a body motion sensor;
a determination unit which determines the exercise state of a subject; and
a control unit which controls at least one of a pulse wave sensing operation in the pulse wave detection unit, a body motion sensing operation in the body motion detection unit, and a calculation processing rate in the pulse information calculation unit on the basis of the determination result of the exercise state in the determination unit.

**20.** A program which causes a computer to function as:

a pulse information calculation unit which calculates pulse information on the basis of a pulse wave detection signal from a pulse wave detection unit having a pulse wave sensor and a body motion detection signal from a body motion detection unit having a body motion sensor;
a determination unit which determines the exercise state of a subject;
a control unit which performs control on the basis of the determination result of the exercise state in the determination unit,
wherein the pulse information calculation unit includes a noise reduction unit which performs noise reduction processing for the pulse wave detection signal, and
the control unit controls the contents of the noise reduction processing in the noise reduction unit on the basis of the determination result of the exercise state.

**Amended claims under Art. 19.1 PCT**

**1.** (Amended)
A pulse detector comprising:

a pulse information calculation unit which calculates pulse information on the basis of a pulse wave detection signal from a pulse wave detection unit having a pulse wave sensor and a body motion detection signal from a body motion detection unit having a body motion sensor;
a determination unit which determines whether a subject is exercising or in living activity; and
a control unit which performs at least two of control for lowering an operation rate of a pulse wave sensing operation, control for lowering an operation rate of a body motion sensing operation, and control for lowering a calculation processing rate when the determination unit determines that the subject is in living activity compared to when it is determined that the subject is exercising.

**2.** (Amended) The pulse detector according to claim 1,
wherein the determination unit determines whether the subject is in living activity or sleeping, and
the control unit performs at least one of control for lowering the operation rate of the pulse wave sensing operation, control for lowering the operation rate of the body motion sensing operation, and control for lowering the calculation processing rate when the determination unit determines that the subject is sleeping compared to when it is determined that the subject is in living activity.

**3.** (Amended) The pulse detector according to claim 1,
wherein the determination unit determines whether the subject is exercising or sleeping, and
the control unit performs at least two of control for lowering the operation rate of the pulse wave sensing operation, control for lowering the operation rate of the body motion sensing operation, and control for lowering the calculation processing rate when the determination unit determines that the subject is sleeping compared to when it is determined that the subject is exercising.

**4.** (Amended) The pulse detector according to any one of claims 1 to 3,
wherein the control unit controls calculation processing contents in the pulse information calculation unit on the basis of the determination result of the exercise state in the determination unit.

**5.** (Amended) The pulse detector according to claim 4,
wherein the pulse information calculation unit includes a noise reduction unit which performs noise reduction processing for the pulse wave detection signal, and
the control unit controls the contents of the noise reduction processing in the noise reduction unit on the basis of the determination result of the exercise state.

**6.** (Amended) The pulse detector according to claim 1,
wherein the determination unit determines whether the subject is exercising or in living activity, and
when the determination unit determines that the subject is in living activity,
the control unit performs control for setting the operation rate of the pulse wave sensing operation to the operation rate whose ratio to the operation rate of the pulse wave sensing operation when it is determined that the subject is exercising is R1 (where R1 is a real number which satisfies 0 < R1 < 1), and

the control unit performs control for setting the operation rate of the body motion sensing operation to the operation rate whose ratio to the operation rate of the body motion sensing operation when it is determined that the subject is exercising is R2 (where R2 is a real number which satisfies $0 < R1 < R2 \leq 1$).

**7.** (Amended)
An electronic apparatus comprising:

the pulse detector according to any one of claims 1 to 6;
the pulse wave detection unit; and
the body motion detection unit.

**8.** (Amended) A program which causes a computer to function as:

a pulse information calculation unit which calculates pulse information on the basis of a pulse wave detection signal from a pulse wave detection unit having a pulse wave sensor and a body motion detection signal from a body motion detection unit having a body motion sensor;
a determination unit which determines whether a subject is exercising or in living activity; and
a control unit which performs at least two of control for lowering an operation rate of a pulse wave sensing operation, control for lowering an operation rate of a body motion sensing operation, and control for lowering a calculation processing rate when the determination unit determines that the subject is in living activity compared to when it is determined that the subject is exercising.

**9.** (Cancelled)

**10.** (Cancelled)

**11.** (Cancelled)

**12.** (Cancelled)

**13.** (Cancelled)

**14.** (Cancelled)

**15.** (Cancelled)

**16.** (Cancelled)

**17.** (Cancelled)

**18.** (Cancelled)

**19.** (Cancelled)

**20.** (Cancelled)

**Statement under Art. 19.1 PCT**

Claim 1 was amended based on Claims 1 and 2 before amendment and matters described in paragraph [0177].
Claim 2 corresponds to Claim 3 before amendment.
Claim 3 was amended based on Claim 4 before amendment and paragraphs [0136] and [0137].
Claim 4 corresponds to Claim 5 before amendment.
Claim 5 corresponds to Claim 6 before amendment.
Claim 6 corresponds to Claim 14 before amendment.
Claim 7 corresponds to Claim 18 before amendment.
Claim 8 was amended based on Claim 19 before amendment and matters described in paragraph [0177].
Claims 9 to 20 were cancelled.

Accordingly, this amendment is the amendment within the scope disclosed in the specification as filed.

In addition, the written opinion was issued pointing out that the inventions according to claims 2 to 4 before amendment have no inventive step based on Cited Document 1 and Cited Document 3 cited in the International Search Report.

Cited Document 1: JP-A-2011-193886
Cited Document 3: JP-A-2000-308639

However, these Cited Documents do not disclosed nor suggest the configuration corresponding to the control unit described in Claim 1 after amendment. In other words, the configuration of "the control unit which performs at least two of control for lowering the operation rate of the pulse wave sensing operation, control for lowering the operation rate of the body motion sensing operation, and control for lowering the calculation processing rate when the determination unit determines that the subject is in living activity compared to when it is determined that the subject is exercising" is not disclosed in these Cited Documents. Therefore, the amendment was made to define the configuration.

FIG. 1

PULSE WAVE DETECTION SIGNAL

PULSE COMPONENT m(n) + BODY MOTION COMPONENT t(n)

115

+

$h \cdot k(n)$

−

RESIDUAL SIGNAL e(n) AFTER ADAPTIVE FILTER E(n) ≅ FILTER OUTPUT PULSE SIGNAL

BODY MOTION DETECTION SIGNAL

k(n)

ADAPTIVE FILTER COEFFICIENT (h)

FIG. 2

PULSE WAVE DETECTION SIGNAL

FIG. 3A

BODY MOTION DETECTION SIGNAL

FIG. 3B

OUTPUT PULSE SIGNAL

FIG. 3C

FIG. 4A

FIG. 4B

**FIG. 5**

PULSE DETECTOR 100

PULSE WAVE DETECTION UNIT 10

PULSE WAVE SENSOR 11

FILTER PROCESSING UNIT 15

A/D CONVERSION UNIT 16

PULSE INFORMATION CALCULATION UNIT 120

NOISE REDUCTION UNIT 123

CONTROL UNIT 150

DETERMINATION UNIT 112

DISPLAY UNIT 70

BODY MOTION DETECTION UNIT 20

ACCELERATION SENSOR 21

PRESSURE SENSOR 22

A/D CONVERSION UNIT 26

123

NOISE REDUCTION UNIT

10

PULSE WAVE
DETECTION UNIT

1231

FIRST
FILTER

1232

SECOND
FILTER

112

DETERMINATION
UNIT

CONTROL
UNIT

150

20

BODY MOTION
DETECTION UNIT

FIG. 6

FIG. 7A

FIG. 7B

| EXERCISE STATE | RATES OF PULSE WAVE SENSING OPERATION AND BODY MOTION SENSING OPERATION | CALCULATION PROCESSING RATE AND DISPLAY RATE |
|---|---|---|
| EXERCISING | ALL TIMES | FOR EVERY 4 SECONDS |
| IN LIVING ACTIVITY | FOR EVERY MINUTE AND WHEN ACCELERATION SENSOR INTERRUPT IS GENERATED | FOR EVERY MINUTE AND WHEN ACCELERATION SENSOR INTERRUPT IS GENERATED |
| SLEEPING | FOR EVERY FOUR MINUTES AND WHEN ACCELERATION SENSOR INTERRUPT IS GENERATED | FOR EVERY FOUR MINUTES AND WHEN ACCELERATION SENSOR INTERRUPT IS GENERATED |

# FIG. 8

| EXERCISE STATE | POWER REDUCTION RATE WHEN EXERCISING IS SET AS 1 | CURRENT CONSUMPTION RELATING TO PULSE WAVE DETECTION |
|---|---|---|
| EXERCISING | UNCHANGED | 4 mA |
| IN LIVING ACTIVITY | ABOUT 73% | ABOUT 1.08 mA |
| SLEEPING | ABOUT 93% | ABOUT 0.295 mA |

# FIG. 9

FIG.10

DETERMINATION UNIT
DURING DETERMINATION
DURING STANDBY

ACCELERATION SENSOR
DURING OPERATION (16Hz)
DURING STANDBY

t (sec)

FIG.11

FIG.12

FIG.13

EP 2 832 289 A1

| TRANSITION DESTINATION / CURRENT | EXERCISING | IN LIVING ACTIVITY | SLEEPING |
|---|---|---|---|
| EXERCISING | —— | N TIMES IN SUCCESSION | —— |
| IN LIVING ACTIVITY | ONCE | —— | P TIMES IN SUCCESSION |
| SLEEPING | ONCE | ONCE | —— |

(N>2, P>2)

## FIG.14A

FOR EVERY SECOND
e (EXERCISING)
l (IN LIVING ACTIVITY)
s (SLEEPING)
ANY INTERMEDIATE RESULT
IS ACQUIRED

## FIG.14B

START

INITIALIZE ～S101

S102

INTERRUPT
IS DETERMINED?

| FIRST 16 Hz INTERRUPT ～S103 | SECOND 16 Hz INTERRUPT ～S104 | 1 Hz INTERRUPT ～S105 |

HALT ～S106

FIG.15

EP 2 832 289 A1

```
                        ┌──────────┐
                        │  START   │
                        └──────────┘
                             │
                             ▼
                    ┌─────────────────┐
                    │   DETERMINE     │ ~S201
                    │ EXERCISE STATE  │
                    └─────────────────┘
                             │        S202
                             ▼
                        ╱─────────╲
                       ╱ STATE IS  ╲
                       ╲ TRANSITED?╱
                        ╲─────────╱
                             │                              NO TRANSITION
     TO EXERCISING           │ TO IN LIVING ACTIVITY    TO SLEEPING
 ┌────────────────┐  ┌────────────────────┐  ┌────────────────────┐
 │ INITIALIZE TIMER│  │  NITIALIZE TIMER   │  │  INITIALIZE TIMER  │
 │ 4-SECOND TIMER ON│ │ 16-SECOND TIMER ON │  │ 16-SECOND TIMER ON │
 │ OTHER TIMERS OFF│~S203│ 1-MINUTE TIMER ON│~S204│ 4-MINUTE TIMER ON│~S205
 └────────────────┘  │ OTHER TIMERS OFF   │  │ OTHER TIMERS OFF   │
                     └────────────────────┘  └────────────────────┘
                             │        S206
                             ▼
                        ╱─────────╲
                        ╲ TIMER?  ╱
                        ╲─────────╱
                             │
 ┌────────────────┐  ┌────────────────┐  ┌──────────────┐  ┌──────────────┐
 │ 4-SECOND TIMER │  │ 16-SECOND TIMER│  │ 1-MINUTE TIMER│ │ 4-MINUTE TIMER│
 │          ~S207 │  │         ~S208  │  │       ~S209   │ │       ~S210   │
 └────────────────┘  └────────────────┘  └──────────────┘  └──────────────┘
                             │
                             ▼
                        ┌──────────┐
                        │   END    │
                        └──────────┘
```

FIG.16

## FIG.17A

START

timer4s←timer4s +1 — S301

timer4s≧4 ? — S302

NO

YES

CALCULATE PULSE RATE — S303

DISPLAY — S304

timer4s←0 — S305

END

## FIG.17B

START

timer16s←timer16s +1 — S401

timer16s≧16 ? — S402

NO

YES

CALCULATE PULSE RATE — S403

DISPLAY — S404

timer16s←0 — S405

INHIBIT SECOND 16 Hz INTERRUPT — S406

16-SECOND TIMER OFF — S407

END

START

timer60s←timer60s +1  ~S501

S502

timer60s≧60？  NO

YES

timer60s←0  ~S503

PERMIT SECOND 16 Hz
INTERRUPT  ~S504

16-SECOND TIMER ON  ~S505

END

FIG.18A

START

timer4m←timer4m +1  ~S601

S602

timer4m≧240？  NO

YES

timer4m←0  ~S603

PERMIT SECOND 16 Hz
INTERRUPT  ~S604

16-SECOND TIMER ON  ~S605

END

FIG.18B

| TIMER ＼ STATE | EXERCISING | IN LIVING ACTIVITY | SLEEPING |
|---|---|---|---|
| 4-SECOND | ○ | × | × |
| 16-SECOND | × | △ | △ |
| 1-MINUTE | × | ○ | × |
| 4-MINUTE | × | × | ○ |

# FIG.19

## INTERNATIONAL SEARCH REPORT

| | International application No. |
|---|---|
| | PCT/JP2013/002063 |

A. CLASSIFICATION OF SUBJECT MATTER
*A61B5/0245*(2006.01)i, *A61B5/0205*(2006.01)i

According to International Patent Classification (IPC) or to both national classification and IPC

B. FIELDS SEARCHED

Minimum documentation searched (classification system followed by classification symbols)
A61B5/0245, A61B5/0205

Documentation searched other than minimum documentation to the extent that such documents are included in the fields searched
| Jitsuyo Shinan Koho | 1922-1996 | Jitsuyo Shinan Toroku Koho | 1996-2013 |
| Kokai Jitsuyo Shinan Koho | 1971-2013 | Toroku Jitsuyo Shinan Koho | 1994-2013 |

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| X | JP 2011-193886 A  (Seiko Epson Corp.), | 1,5,6,17-20 |
| Y | 06 October 2011 (06.10.2011), paragraphs [0032] to [0034], [0039] to [0049] (Family: none) | 2-4,7-14 |
| X | JP 2011-98002 A  (Seiko Epson Corp.), 19 May 2011 (19.05.2011), paragraphs [0017] to [0031]; fig. 5 to 8 (Family: none) | 1,5,6,15-20 |
| Y | JP 2000-308639 A  (Seiko Instruments Inc.), 07 November 2000 (07.11.2000), paragraphs [0006] to [0008], [0015] to [0016] (Family: none) | 2-4,14 |

☒ Further documents are listed in the continuation of Box C.   ☐ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 09 May, 2013 (09.05.13) | 21 May, 2013 (21.05.13) |

| Name and mailing address of the ISA/ | Authorized officer |
|---|---|
| Japanese Patent Office | |
| Facsimile No. | Telephone No. |

Form PCT/ISA/210 (second sheet) (July 2009)

**INTERNATIONAL SEARCH REPORT**

| International application No. |
| --- |
| PCT/JP2013/002063 |

C (Continuation).   DOCUMENTS CONSIDERED TO BE RELEVANT

| Category* | Citation of document, with indication, where appropriate, of the relevant passages | Relevant to claim No. |
| --- | --- | --- |
| Y | JP 2011-92236 A  (Seiko Epson Corp.),<br>12 May 2011 (12.05.2011),<br>paragraphs [0013] to [0015]<br>& US 2011/0098582 A1 | 7 |
| Y | JP 2006-288970 A  (Matsushita Electric Works, Ltd.),<br>26 October 2006 (26.10.2006),<br>paragraphs [0026] to [0035]<br>(Family: none) | 8-13 |
| Y | JP 2006-101973 A  (Microstone Kabushiki Kaisha),<br>20 April 2006 (20.04.2006),<br>paragraph [0051]<br>(Family: none) | 13 |
| A | JP 8-317912 A  (Seiko Instruments Inc.),<br>03 December 1996 (03.12.1996),<br>entire text; all drawings<br>& US 5749366 A          & EP 733340 A1<br>& DE 69634147 D | 1-20 |
| A | JP 2002-42280 A  (Seiko Precision Inc.),<br>08 February 2002 (08.02.2002),<br>entire text; all drawings<br>(Family: none) | 1-20 |
| A | JP 2008-246179 A  (Matsushita Electric Works, Ltd.),<br>16 October 2008 (16.10.2008),<br>entire text; all drawings<br>(Family: none) | 1-20 |
| A | JP 2010-539617 A  (Koninklijke Philips Electronics N.V.),<br>16 December 2010 (16.12.2010),<br>entire text; all drawings<br>& US 2010/0261980 A1      & EP 2203910 A<br>& WO 2009/037612 A2      & CN 101802881 A | 1-20 |

Form PCT/ISA/210 (continuation of second sheet) (July 2009)

## INTERNATIONAL SEARCH REPORT

| International application No. |
| --- |
| PCT/JP2013/002063 |

**Box No. II      Observations where certain claims were found unsearchable (Continuation of item 2 of first sheet)**

This international search report has not been established in respect of certain claims under Article 17(2)(a) for the following reasons:

1. ☐ Claims Nos.:
   because they relate to subject matter not required to be searched by this Authority, namely:

2. ☐ Claims Nos.:
   because they relate to parts of the international application that do not comply with the prescribed requirements to such an extent that no meaningful international search can be carried out, specifically:

3. ☐ Claims Nos.:
   because they are dependent claims and are not drafted in accordance with the second and third sentences of Rule 6.4(a).

**Box No. III      Observations where unity of invention is lacking (Continuation of item 3 of first sheet)**

This International Searching Authority found multiple inventions in this international application, as follows:
      See extra sheet.

1. ☐ As all required additional search fees were timely paid by the applicant, this international search report covers all searchable claims.

2. ☒ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☐ No required additional search fees were timely paid by the applicant.   Consequently, this international search report is restricted to the invention first mentioned in the claims; it is covered by claims Nos.:

**Remark on Protest**      ☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet (2)) (July 2009)

**INTERNATIONAL SEARCH REPORT**

International application No.

PCT/JP2013/002063

Continuation of Box No.III of continuation of first sheet(2)

Document 1 (JP 2011-193886 A (Seiko Epson Corp.), 06 October 2011 (06.10.2011), paragraphs [0032] to [0034], [0039] to [0049]) discloses the invention of a pulse wave measurement device comprising a pulse wave sensor and a body motion sensor, wherein a mode is switched by body motion data to switch a filtering process and a measuring operation of the pulse wave sensor.

Therefore, the invention of claim 1 cannot be considered to be novel in the light of the invention disclosed in the document 1, and does not have a special technical feature.

Consequently, the following four inventions (invention groups) are involved in claims.

Meanwhile, the invention of claim 1 having no special technical feature is classified into invention 1.

(Invention 1) Claims 1-6, 14, 17-20: A pulsation detection device for controlling any one of an operation rate of a pulse wave sensing operation, an operation rate of a body motion sensing operation, and a computing process rate based on a determination result of exercise states.

(Invention 2) Claim 7: A pulsation detection device including a first filter for performing an enhancement filer process, and a second filter for performing a noise reduction process.

(Invention 3) Claims 8-13: A pulsation detection device for determining an exercise state based on a body motion detection signal in an exercise state determination period.

(Invention 4) Claims 15, 16: A pulsation detection device including a body motion detection unit including an exercise state determination sensor and a body motion noise reduction sensor.

Form PCT/ISA/210 (extra sheet) (July 2009)

**REFERENCES CITED IN THE DESCRIPTION**

**Patent documents cited in the description**

- JP 60259239 A


**Non-patent literature cited in the description**

- Exercise Guide for Health Promotion 2006 - lifestyle-related disease prevention. Exercise Guide. Ministry of Health, Labour and Welfare, 2006